# EUROPÄISCHE PATENTANMELDUNG

(11) **EP 4 617 659 A1**
(43) Veröffentlichungstag der Anmeldung: **17.09.2025**
(21) Anmeldenummer: 25163193.3
(22) Anmeldetag: 12.03.2025
(51) Int. Cl.: G01N 33/00, G01N 27/02

(54) **VORRICHTUNG ZUM AUTOMATISIERTEN SAMMELN VON MESSDATEN FÜR SENSORPARAMETER EINES GASSENSORS**

(30) Priorität: 14.03.2024 DE 102024107305
(71) Anmelder: Hochschule Trier, Körperschaft des öffentlichen Rechts, 54293 Trier (DE)
(72) Erfinder: Dartmann, Guido, 55768 Hoppstädten-Weiersbach (DE); Junk, David, 55768 Hoppstädten-Weiersbach (DE); Haab, Swen, 55768 Hoppstädten-Weiersbach (DE); Gollmer, Klaus-Uwe, 55768 Hoppstädten-Weiersbach (DE); Czenkusch, Levin, 55768 Hoppstädten-Weiersbach (DE)
(74) Vertreter: Durm Patentanwälte PartG mbB

(57) **Zusammenfassung**

Die vorliegende Erfindung betrifft eine Vorrichtung (10) zum automatisierten Durchführen von Messungen und Sammeln von Messdaten und zum Erzeugen wenigstens eines optimierten Sensorparameters für einen Metalloxid-Sensor (23) und/oder einer optimierten Analysestrategie einer Auswerteeinheit (70) zur Bestimmung eines in einer bekannten Probe enthaltenen Stoffs. Die Vorrichtung (10) umfasst einen Sensorkopf (20) mit wenigstens einem Sensor (22), der mit Sensorparametern angesteuert wird, zwei oder mehrere Gefäße für zu untersuchende Proben oder Flüssigkeiten, eine Bewegungseinheit (40) zum Erzielen einer Messposition, in der der Sensorkopf (20) oberhalb eines der Gefäße angeordnet ist, eine Steuereinheit (50) zum Ansteuern des Sensors (22) mit einem Sensorparameter und eine Auswerteeinheit (70) zum Auswerten der von dem Sensor gemessenen Messdaten. Die vorliegende Erfindung betrifft ferner ein entsprechendes Verfahren sowie ein Speichermedium und eine KI-Einheit (80).

## Beschreibung

Die vorliegende Erfindung betrifft eine Vorrichtung zum automatisierten Durchführen von Messungen und Sammeln von Messdaten und zum Erzeugen wenigstens eines optimierten Sensorparameters für einen Metalloxid-Sensor und/oder einer optimierten Analysestrategie einer Auswerteeinheit zur Bestimmung eines in einer bekannten Probe enthaltenen Stoffs. Die Vorrichtung umfasst einen Sensorkopf mit wenigstens einem Sensor, der mit Sensorparametern angesteuert wird, zwei oder mehrere Gefäße für zu untersuchende Proben oder Flüssigkeiten, eine Bewegungseinheit zum Erzielen einer Messposition, in der der Sensorkopf oberhalb eines der Gefäße angeordnet ist, eine Steuereinheit zum Ansteuern des Sensors mit einem Sensorparameter und eine Auswerteeinheit zum Auswerten der von dem Sensor gemessenen Messdaten.

Künstliche Nasen oder elektronische Nasen gewinnen in der Forschung und Industrie in vielen Bereichen zunehmend an Bedeutung. Die Idee, die menschliche Nase mit einem elektronischen Instrument nachzuahmen, um Gerüche zu erkennen, wird beispielsweise in der Lebensmittelindustrie eingesetzt und erprobt. Die elektronische Nase in Form eines Gassensors kann sowohl in der Qualitätskontrolle als auch bei Herstellungsprozessen zur Unterstützung eingesetzt werden. Dabei sollen preiswerte Sensoren zum Einsatz kommen, um einzelne Parameter oder Stoffe in einer Flüssigkeit zu erkennen und zu detektieren.

Die US 2019 0 302 068 A1 beschreibt ein Gaschromatographiesystem und einen Autosampler für mehrere Probenhalter. Probleme beim Entweichen von Gasen adressiert die EP 0 841 094 B1, die Probenfläschchen und eine Verschlussvorrichtung zur Verwendung in der Gasprobennahme beschreibt.

Ein mögliches Einsatzgebiet von Metalloxyd-Sensoren ist in der Lebensmittelindustrie. Beispielsweise kann ein in einem Handgerät integrierter Sensor zur Unterstützung des Handels bei der Qualitätsbestimmung von Lebensmitteln eingesetzt werden. Aus der WO 2003 027 667 A1 ist ein Verfahren und ein Detektor zur Bestimmung von Gasen bekannt.

Für bestimmte Anwendungen sind spezifisch ausgestattete Metalloxyd-Sensoren bekannt. Eine Sensorart wird beispielsweise beschrieben in "Thermocyclically operated metal oxide gas sensor arrays for analysis of dissolved volatile organic compounds in fermentation processes, Part I Morphology aspects of the sensing behavior"; OJHA, Binayak [et al.]; Sensing and Bio-Sensing Research, Vol. 40, 2023, S. 1-20 (100558). ISSN 2214-1804. DOI: https://doi.org/10.1016/j.sbsr.2023.100558.

Aus der WO 2003 027 667 A1 ist ein Verfahren und ein Detektor zur Bestimmung von Gasen bekannt.

Die Veröffentlichung von STANKOVA, M. [et al.]: Sputtered and screen-printed metal oxide-based integrated micro-sensor arrays for the quantitative analysis of gas mixtures. In: Sensors and Actuators B, Vol. 103, 2004, No. 1-2, S. 23-30. ISSN 0925-4005. DOI: https://doi.org/10.1016/j.snb.2004.02.022 beschreibt eine Untersuchung zur Herstellung neuartiger Sensoren, um bestimmte Gase identifizieren zu können.

In der Veröffentlichung von GHERMAN, Markus-Philipp [et al.]: Compensating altered sensitivity of duty-cycled MOX gas sensors with machine learning. 2021 18th Annual IEEE International Conference on Sensing, Communication, and Networking (SECON), 6-9 July 2021, Virtual. Piscataway, New Jersey, USA: IEEE, 2021. S. 1-9. ISBN 978-1-6654-4108-7. DOI: https://doi.orq/10.1109/SECON52354.2021.9491586 wird gezeigt, dass transient verwendete Gassensoren zur Reduktion des Energieverbrauchs abweichende Ergebnisse von kontinuierlich verwendeten Gassensoren zeigen. Insbesondere für neuere Sensoren gibt es nur wenige bis keine Erfahrungswerte, für welche Anwendungsfälle und Stoffe in Flüssigkeiten der Sensor besonders gut geeignet ist. Derartige Sensoren werden mit einem dynamischen Temperaturbetrieb angesteuert. Dabei wird die Sensoroberfläche nacheinander auf unterschiedliche, vorher definierte Temperaturstufen erhitzt, die in einem Temperatur- oder Heizprofil abgelegt sein können. Die Detektion von Gasen bzw. von Stoffen in einer Flüssigkeit oder in einem Festkörper ist abhängig von dem Temperaturprofil, d. h. ein Sensor reagiert für jedes Temperaturprofil unterschiedlich auf einen vorhandenen Stoff. Daher muss für jeden Anwendungsfall ein passendes Profil für den Sensor gefunden werden.

Gassensoren verfügen also über verschiedene, einstellbare Parameter, welche das Messergebnis beeinflussen. Um einen spezifischen Stoff zu detektieren, ist es notwendig, die richtigen Einstellungen der Parameter für die Sensoren zu verwenden. Die Einstellung der Parameter ist jedoch mitunter nicht trivial, sondern aufwendig und muss erst durch eine große Anzahl von Versuchen herausgefunden werden.

Es ist Aufgabe der vorliegenden Erfindung, eine verbesserte Vorrichtung zum Sammeln von Sensorparametern eines Gassensors zur Bestimmung eines Stoffs in einer Probe, einer Flüssigkeit oder einem Festkörper vorzuschlagen, die eine schnellere und qualitativ hochwertigere Einstellung von Metalloxid-Sensoren erlaubt.

Gelöst wird die vorliegende Aufgabe mit einer Vorrichtung mit den Merkmalen des Anspruchs 1, einem Verfahren mit den Merkmalen des Anspruchs 11 sowie einer KI-Einheit mit den Merkmalen des Anspruchs 14.

In einem ersten Aspekt betrifft die vorliegende Erfindung eine Vorrichtung zum automatisierten Durchführen von Messungen und Sammeln von Messdaten und zum Erzeugen wenigstens eines optimierten Sensorparameters für einen Metalloxid-Sensor und/oder einer optimierten Analysestrategie einer Auswerteeinheit zur Bestimmung eines in einer bekannten Probe enthaltenen Stoffs, mit einem Sensorkopf mit wenigstens einem Metalloxid-Sensor, zwei oder mehreren Probegefäßen, einer Bewegungseinheit zum Bewegen des Sensorkopfs und/oder eines Probengefäßes, einer Steuereinheit zum Ansteuern des Metalloxid-Sensors, einer Auswerteeinheit zum Auswerten von Messdaten und einer KI-Einheit.

Der Metalloxid-Sensor des Sensorkopfs wird mit mindestens einem Sensorparameter angesteuert, der den Metalloxid-Sensor zum Erfassen eines oder mehrerer spezifischer Stoffe in der Probe konfiguriert. Die Probe kann ein Feststoff, ein Festkörper, eine Flüssigkeit und/oder ein Gas sein, wobei der gesuchte Stoff darin enthalten ist. Die Bewegungseinheit ist dazu ausgebildet, den Sensorkopf und/oder ein Probengefäß in einer Messposition zu bewegen, in der der Sensorkopf oberhalb oder teilweise innerhalb eines der Probengefäße angeordnet ist. Der Sensorkopf und/oder der Metalloxid-Sensor können dabei folglich mindestens teilweise in das Probengefäß hineinragen.

Die Steuereinheit ist zum Ansteuern des Metalloxid-Sensors mit wenigstens einem Sensorparameter ausgebildet. Es versteht sich, dass auch mehrere individuelle Parameter zum Ansteuern des Metalloxid-Sensors Anwendung finden können.

Die Auswerteeinheit ist eingerichtet, um die vom Metalloxid-Sensor gemessenen Messdaten auszuwerten. Hierfür kann sie sich einer Analysestrategie bedienen, die eine oder mehrere bekannte Datenaufbereitungsmethoden und/oder Merkmalsextraktionsmethoden oder dergleichen umfasst.

Die KI-Einheit ist dazu ausgebildet, einen Sensorparameter anzupassen und/oder zu optimieren, basierend auf der bekannten Probe und aktuellen Messdaten sowie dem zur Erhebung der Messdaten verwendeten Sensorparameter.

Die KI-Einheit ist ferner dazu ausgebildet, einen auf den Messdaten und den Sensorparametern basierenden Fitnesswert zu ermitteln, vorzugsweise daraus Rückschlüsse zwischen den Messdaten und dem Sensorparameter zu ziehen und eine Optimierung des Sensorparameters basierend auf den Messdaten und dem Fitnesswert durchzuführen. Hierbei kann mittels der KI-Einheit eine schnelle Anpassung und/oder Optimierung des Sensors und/oder anderer Sensoren erfolgen, insbesondere eine schnellere Anpassung als dies von einem Anwender möglich wäre. Die Anpassung und/oder Optimierung des Sensorparameters kann vorzugsweise während der Messung oder nach einem Messzyklus erfolgen.

Die KI-Einheit ist weiter dazu ausgebildet, aus den Messdaten früherer Messungen sowie den aktuellen Messdaten und/oder den verwendeten Sensorparametern einen verbesserten Sensorparameter für den Metalloxid-Sensor zu generieren und/oder eine verbesserte Analysestrategie für die Auswerteeinheit zu bestimmen. Die KI-Einheit wird in die Lage versetzt, aus der Anpassung zu lernen und beispielsweise Trainingsdaten für den Sensor oder andere Sensoren oder für die Auswerteeinheit oder andere Auswerteeinheiten zu erstellen.

In einem weiteren Aspekt betrifft die Erfindung ein Verfahren zum automatisierten Durchführen von Messungen und Sammeln von Messdaten und zum Erzeugen von optimalen Sensorparametern eines Metalloxid-Sensorsund/oder einer optimierten Analysestrategie einer Auswerteeinheit zur Bestimmung eines in einer Probe enthaltenen Stoffs oder zur Bestimmung von Trainingsdaten eines derartigen Metalloxid-Sensors und/oder einer derartigen Auswerteeinheit, umfassend die folgenden Schritte: Bewegen eines Sensorkopfs mit einem Metalloxid-Sensor und/oder eines Probengefäßes für die zu vermessende Probe in eine Messposition, in der der Sensorkopf oberhalb des Probengefäßes angeordnet ist; Auswählen eines initialen, vorbestimmten Sensorparameters; Ansteuern des Metalloxid-Sensors mit dem vorbestimmten Sensorparameter; Messen eines Stoffs, der in der Probe in dem Probengefäß enthalten ist; Aufnehmen von Messdaten mit dem Metalloxid-Sensor und Auswerten der aufgenommenen Messdaten; Aufbereiten der Messdaten, wobei vorzugsweise eine Datenbereinigung, Datentransformation, Datenkorrektur und Berücksichtigung von Umgebungsdaten und/oder eine Dimensionalitätsreduktion erfolgt; Ermittlung von Korrelationen zwischen den Messdaten und den Sensorparametern und dem Stoff der zu vermessenden Probe unter Anwendung einer Auswertungsmethode, die vorzugsweise eine Regressionsmethode und/oder eine Diskriminanzanalyse umfasst; Optimieren der Sensorparameter basierend auf den aufbereiteten Messdaten, um eine schnellere Anpassung des Metalloxid-Sensors oder anderer Metalloxid-Sensoren zu ermöglichen; ergänzend oder alternativ Ermitteln von Korrelation zwischen den Messdaten und der Analysestrategie und dem Stoff der zu vermessenden Probe unter Anwendung einer Auswertungsmethode; ergänzend oder alternativ Optimieren der Analysestrategie basierend auf den aufbereiteten Messdaten, um eine schnellere Anpassung der Auswerteeinheit oder anderer Auswerteeinheiten zu ermöglichen.

Die Aufbereitung und Auswertung der Messdaten und die Anpassung und Optimierung der Sensorparameter und/oder der Analysestrategie kann während des Messbetriebs erfolgen und wird wenigstens teilweise von einer KI-Einheit durchgeführt, die dazu ausgebildet ist, aus einer vorhergegangenen Anpassung zu lernen und vorzugsweise dazu ausgebildet ist, Trainingsdaten für andere Metalloxid-Sensoren und/oder andere Auswerteeinheiten zu erstellen.

Die Optimierung kann auf mehreren Messungen mehrerer Proben basieren, wobei die Optimierung beendet wird, wenn ein vorgegebenes Abbruchkriterium, also beispielsweise eine Anzahl von Maximaldurchläufen, oder ein entsprechendes Gütekriterium erfüllt ist.

Die Vorrichtung, insbesondere die Auswerteeinheit oder die KI-Einheit und/oder das Verfahren können auch dazu geeignet sein, eine Klassifikation von mindestens zwei unterschiedlichen Proben durchzuführen. Dies ist möglich, da der Sensor bevorzugt mehrere Stoffe in der Probe gleichzeitig oder zeitlich versetzt oder nacheinander, bevorzugt direkt hintereinander, messen kann.

Der Sensorparameter zur Steuerung des ganzen Sensors spiegelt dabei bevorzugt die Abhängigkeit der Messdaten und einer gesuchten Klassifikation von, bevorzugt zwei, unterschiedlichen Proben oder Stoffen in Proben wider.

Weitere Aspekte der Erfindung betreffen ein entsprechendes Computerprogrammprodukt mit Programmcode zum Durchführen der Schritte des Verfahrens, wenn der Programmcode auf einem Computer ausgeführt wird, sowie ein Speichermedium, auf dem ein Computerprogramm gespeichert ist, das, wenn es auf einem Computer ausgeführt wird, eine Ausführung des hierin beschriebenen Verfahrens bewirkt.

Weitere Aspekte sind ein Speichermedium zur Verwendung in einer Steuereinheit zum Steuern eines Metalloxid-Sensors, wobei ein mittels einer Vorrichtung wie zuvor beschrieben oder einem Verfahren wie zuvor beschrieben optimierter Sensorparameter auf dem Speichermedium gespeichert ist und das Speichermedium zur Steuerung des Metalloxid-Sensorsmittels der Steuereinheit angewandt wird. Alternativ kann auch das Speichermedium zur Verwendung in einer Auswerteeinheit vorgesehen sein, wobei in analoger Weise eine optimierte Analysestrategie auf dem Speichermedium hinterlegt ist und das Speichermedium zur Verwendung mit einer Auswerteeinheit vorgesehen ist.

Bevorzugte Ausgestaltungen der Erfindung werden in den abhängigen Ansprüchen beschrieben. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Insbesondere können das Verfahren und das Computerprogrammprodukt entsprechend der für die Vorrichtung in den abhängigen Ansprüchen beschriebenen Ausgestaltungen ausgeführt sein.

Im Folgenden werden die Begriffe Sensor und Gassensor sowie Metalloxid-Gassensor und Metalloxid-Sensor synonym verwendet. Die Erfindung ist auf derartige Metalloxid-Sensoren gerichtet, die Gase messen. Die im Folgenden beschriebenen Proben schließen Flüssigkeiten mit ein. Sie umfassen auch gasförmige oder feste Proben, also Feststoffe. Dabei werden jeweils die austretenden Gase detektiert.

Metalloxid-Sensoren sind Halbleitersensoren, die häufig zum Erkennen von giftigen Gasen oder zur Bestimmung der Luftqualität durch Messung flüchtiger organischer Verbindungen eingesetzt werden. Derartige Sensoren sind preiswert, einfach herstellbar und für eine Vielzahl von Anwendungen geeignet. Allerdings weisen sie in der Regel eine schlechte Selektivität, eine auftretende Kreuzselektivität bei mehreren vorhandenen Stoffen und eine geringe Empfindlichkeit gegenüber niedrigen Gaskonzentrationen auf.

Metalloxid-Sensoren haben eine auf einem Substrat aufgebrachte Halbleiterschicht, die heizbar ist. Zwischen zwei Elektroden entsteht ein Stromfluss aus freien Elektroden im Halbleiterelement, wobei eine Reaktion mit Sauerstoff an der Halbleiteroberfläche erfolgt, wenn der Sensor in Sauerstoff oder (reiner) Luft beaufschlagt wird. Dies führt zu einer Reduzierung der Leitfähigkeit und des Stromflusses. Sind in dem Messgas Gase umfasst, die mit Sauerstoff reagieren, so führt dies ebenfalls zur Änderung der Leitfähigkeit und somit des Widerstands. Die an der Oberfläche stattfindenden Reaktionen sind von der Oberflächentemperatur des Sensors abhängig, ebenso wie der Widerstandswert des Sensors. Das Betreiben des Sensors kann dabei mit einer konstanten Temperatur oder mit zeitlich veränderlichen Temperaturen erfolgen.

Im Rahmen der Erfindung wurde erkannt, dass für unterschiedliche Stoffe in den zu vermessenden Proben die Leitwerte des Sensors bzw. die Widerstandswerte temperaturspezifisch sind. Unterschiedliche Gase haben unterschiedliche optimale Oxidationstemperaturen, sodass mehrere Gase in der Probe detektiert und gemessen werden können. Dieser Effekt tritt besonders bei der Ansteuerung des Sensors mit, bevorzugt zyklischen, Temperaturänderungen der Heiztemperatur des Sensors auf. Die Temperaturen können dabei im Bereich von ca. 200 °C bis ca. 400 °C liegen.

Es wurde weiter erkannt, dass ein temperaturzyklischer Betrieb des Sensors nicht nur das Erkennen mehrerer Gase ermöglicht, sondern auch die Empfindlichkeit des Sensors steigert. Dabei ist es von Vorteil, wenn Sensorparameter zum Ansteuern des Sensors gesammelt werden können, um Messungen mit hoher Qualität, Güte und Schnelligkeit auch zu einem späteren Zeitpunkt, in veränderten Umgebungen oder mit abweichenden, aber ähnlichen oder bau- oder typgleichen Sensoren durchführen zu können. Dies bietet die Möglichkeit, recht einfache und kostengünstige Sensoren zu verwenden.

Erfindungsgemäß wird ein automatisiertes Sammeln von Sensorparametern ermöglicht, bei dem mittels eines Metalloxid-Gassensors ein Stoff in einer zu untersuchenden Probe oder Flüssigkeit detektiert wird. Dabei misst der Metalloxid-Gassensor ein aus der Probe austretendes Gas, das spezifisch für den Stoff in der Probe ist. Um die Sensibilität für einen bestimmten Stoff zu erhöhen, müssen geeignete Sensorparameter verwendet werden, die den Metalloxid-Gassensor für die spezifischen Stoffe konfigurieren. Die Anpassung und Optimierung der Sensorparameter wird von einer KI-Einheit unterstützt, die dazu ausgebildet ist, aus den durchgeführten Anpassungen zu lernen und so Trainingsparameter für andere, bevorzugt ähnliche oder typgleiche Sensoren zu erstellen.

Hierfür ist eine Vielzahl von Daten notwendig, die mittels der automatisierten Vorrichtung erzeugt werden können, da automatisch eine große Anzahl von Messungen und eine Anpassung der Sensorparameter durchgeführt werden kann. In der Regel werden mehrere hundert oder tausend Datensätze benötigt, um eine Optimierung der Trainingsdaten und eine Optimierung der Sensordaten bzw. Sensorparameter vornehmen zu können.

Mittels der Bewegungseinheit wird eine Messposition von Sensorkopf zu Probengefäß mit der zu vermessenden Probe angefahren, wobei der Sensorkopf oder das Probengefäß oder beide, relativ zueinander, bewegt werden können. Somit lassen sich Messungen an Flüssigkeiten in mehreren Probengefäßen schnell und automatisch durchführen. Es lassen sich deshalb die Sensorparameter zur Ansteuerung des Metalloxid-Gassensors auf einfache und zeitlich optimierte Weise ermitteln. Die Bewegungseinheit kann einen oder mehrere Aktoren umfassen, um die Bewegung auszuführen. Beispielsweise können Schrittmotoren und eine entsprechende Mechanik verwendet werden. Somit lassen sich mehrere Probengefäße nacheinander anfahren und mehrere Proben untersuchen bzw. den oder die Sensoren mit mehreren Proben trainieren, die unterschiedlich sein können. Bei mehreren gleichen Proben lassen sich unterschiedliche Komponenten oder Inhaltsstoffe für eine Optimierung der Sensoren verwenden, um die Sensitivität auf unterschiedliche Inhaltsstoffe zu verbessern.

Aus den ermittelten Messwerten und den dazugehörigen Sensorparametern werden optimierte und für einen zu ermittelnden Stoff spezifische Trainingsdaten mittels einer KI-Einheit ermittelt, mit denen Metalloxid-Sensoren gleichen Typs oder gleicher Bauart oder ähnliche Sensoren trainiert werden können. Das Lernen der KI-Einheit aus der Anpassung kann dabei auf spezifischen oder gängigen Lern- oder Trainingsalgorithmen beruhen. Hier können Ansätze für maschinelles Lernen zum Einsatz kommen.

In einer bevorzugten Ausgestaltung der Vorrichtung ist eine Heizplatte vorgesehen, die wenigstens eines der Probengefäße beheizt. Beispielsweise kann das Probengefäß in der Messposition geheizt werden. Bevorzugt kann die Heizplatte unterhalb des Probengefäßes angeordnet sein. Sie kann jedoch auch in Form eines Heizmantels ausgebildet sein und das Probengefäß teilweise umschließen. Besonders bevorzugt ist eine Ausführungsform, bei der eine oder mehrere Heizplatten oder -matten vorgesehen sind. Bevorzugt wird jedes Probengefäß mit einer einzelnen Heizplatte versehen, sodass jedes der Gefäße separat beheizt werden kann. Die Heizleistung der Heizplatte ist als Sensorparameter mittels der KI-Einheit optimierbar.

In einer bevorzugten Ausführungsform der Vorrichtung wird ein Sensorkopf verwendet, der mehrere Sensoren umfasst. Hierbei können zwei, vier oder mehr Sensoren vorgesehen sein. Auch ist es möglich, eine große Gruppe von Sensoren auf einer einzelnen Platine zu platzieren. Ebenso bevorzugt ist ein Sensorkopf, der mehrere Platinen mit Sensoren aufnehmen kann. Besonders bevorzugt können die Sensoren des Sensorkopfs gleichzeitig oder zeitversetzt, mit oder ohne zeitliche Überlappung, messen. Somit können unterschiedliche Zeitpunkte des Beginns der Messung festgelegt werden. Auch ist es möglich, die einzelnen Sensoren zu gruppieren und parallel messen zu lassen. Bei einer gleichzeitigen, ungruppierten oder zeitversetzten Messung ist es möglich, mit mehreren Sensoren gleichzeitig mehrere Stoffe in der Probe oder mehrere Gase in der Probe zu detektieren. Darüber hinaus ist es auch möglich, unterschiedliche Sensortypen oder baugleiche Sensoren miteinander zu vergleichen und auf einfache Art eine große Anzahl an Messdaten zu gewinnen. Hierdurch werden die Messung, die Optimierung und Anpassung beschleunigt.

In einer bevorzugten Ausgestaltung nimmt die KI-Einheit eine optimierte Anpassung der Sensorparameter vor, wobei die Anpassung durch Auswahl vorhandener und passender Sensorparameter aus einem Sensorparametersatz oder einer Datenbank oder durch Erzeugen neuer Sensorparameter erfolgt. Bevorzugt erfolgt die Anpassung basierend auf vorhergehenden Anpassungen eines vorhergehenden Messzyklus oder einer vorherigen Messung oder auf Anpassungen von Sensorparametern von Sensoren für andere Stoffe. In anderen Worten kann die KI-Einheit aus vergangenen Messungen oder einem Sensorparametersatz schöpfen, um so eine schnelle Optimierung wenigstens eines Sensorparameters zu erreichen. Hierdurch kann einerseits eine Zeitersparnis beim Einrichten und Optimieren eines Sensorparameters erreicht werden und zudem können Betriebskosten für eine derartige Vorrichtung geringgehalten werden.

In einer besonders bevorzugten Ausgestaltung ist die Steuereinheit dazu ausgebildet, den Sensor mittels eines Sensorparameters in Form einer Temperaturkurve, einer Temperatur und/oder einer Dauer einer Aufheizphase bis zum Erreichen einer vorgegebenen Temperatur anzusteuern. Bevorzugt umfasst der Sensorparameter zusätzlich Informationen zu der Umgebung, dem Sensortyp, zu Haltezeiten für bestimmte Temperaturen, zu Abkühlphasen oder Proben oder die Messdauer je Probe.

In einer vorteilhaften Ausgestaltung ist die KI-Einheit dazu ausgebildet, einen Fitnesswert, basierend auf einem Trennbarkeitswert, auf von der Auswerteeinheit ausgewerteten Messdaten und vorzugsweise auf Referenzdaten zu berechnen. Die ausgewerteten Messdaten werden aus den Messdaten mittels einer Analysestrategie, insbesondere bevorzugt umfassend eine Datenaufbereitung, eine Merkmalsextraktion und/oder -reduktion, Transformation und/oder Klassifikation gewonnen. Die Referenzdaten werden aus einer Referenzmessung oder aus einer Referenzdatenbank mit sensortypspezifischen Referenzdaten erzeugt. Durch einen Fitnesswert kann ein zuverlässiges Kriterium geschaffen werden, um einen optimierten Sensorparameter und/oder eine optimierte Analysestrategie bezüglich ihrer Wirksamkeit zu bewerten. Anhand des Fitnesswerts, der beispielsweise eine Trennbarkeit einzelner Proben umfassen kann, kann zudem eine Güte der Messung mittels des Sensors bei Anwendung des optimierten Sensorparameters oder einer Auswertung mittels der Auswerteeinheit bei Auswertung einer optimierten Analysestrategie getroffen werden.

In besonders bevorzugter Ausgestaltung führt die KI-Einheit die Anpassung der Sensorparameter und/oder die Anpassung der Analysestrategie mittels eines evolutionären Lernens, mittels eines bestärkenden Lernens, mittels Entscheidungsbäumen und/oder mittels eines künstlichen neuronalen Netzes durch. Die KI-Einheit bedient sich somit einer Vielzahl von möglichen Algorithmen und Verfahren zur Optimierung der Sensorparameter und/oder der Analysestrategie. Folglich kann eine hochflexible und variabel einsetzbare KI-Einheit geschaffen werden, die sich für verschiedene Auswerteeinheiten und/oder Sensortypen eignet und optimal auf die jeweilige Vorrichtung angepasst sein kann.

In besonders bevorzugter Ausgestaltung ist die KI-Einheit dazu ausgebildet, Rückschlüsse aus Abhängigkeiten zwischen Temperatursteuerkurve, Sensortyp, Heiztemperatur, Arbeitstemperatur, zu messender Probe, umfassten Stoffen in der Probe, Analysestrategie und/oder optional Temperatur der Heizplatte zu ziehen, um einen optimierten Sensorparameter zur Steuerung des Metalloxid-Sensors oder für andere Metalloxid-Sensoren und/oder eine optimierte Analysestrategie für die Auswerteeinheit oder andere Auswerteeinheiten zu generieren. Hierdurch kann eine KI-Einheit geschaffen werden, die mit zunehmender Betriebsdauer eine erhöhte Effizienz und verbesserte Optimierungen erlaubt. Es kann eine KI-Einheit geschaffen werden, die sich stetig verbessert. Besonders bevorzugt kann die KI-Einheit dazu ausgebildet sein, ihre Erfahrung mit anderen KI-Einheiten auszutauschen, vorzugsweise mittels im Stand der Technik bekannter Kommunikationsverfahren.

In besonders bevorzugter Ausgestaltung sind der Sensorkopf, die zwei oder mehreren Probengefäße und die Bewegungseinheit in einem Gehäuse mit einer verschließbaren Öffnung angeordnet, um die Messungen in definierter Umgebung auszuführen. Ein Öffnungsgrad der Öffnung ist mittels der Steuereinheit einstellbar und besonders bevorzugt mittels der KI-Einheit als Sensorparameter optimierbar. Hierdurch kann ein weiterer Sensorparameter geschaffen werden, der durch Optimierung mittels der KI-Einheit zu einem besseren Messergebnis führen kann.

Besonders bevorzugt ist die Steuereinheit dazu ausgebildet, vor einer Messung einer Probe, eine Referenzmessung mit Luft, Reinluft, Umgebungsluft oder einer Referenzprobe durchzuführen. Hierdurch kann die Präzision bei der Messung erhöht werden. Insbesondere kann wirksam ausgeschlossen werden, dass Restwerte von vergangenen Messungen die aktuelle Messung negativ beeinflussen.

In besonders bevorzugter Ausgestaltung ist die Vorrichtung mobil ausgestaltet und dazu eingerichtet, Metalloxid-Sensoren in Einsatzumgebungen für die jeweilige Anwendung optimal einzustellen und die Einflüsse der jeweiligen Einsatzumgebung widerzuspiegeln, wobei bevorzugt die Vorrichtung eine Energieversorgungseinheit umfasst, um die mobile Vorrichtung an eine vorhandene Energieversorgung anzuschließen oder autark zu betreiben. In analoger Weise kann auch eine Optimierung der Analysestrategie vor Ort erfolgen. Durch die mobile Ausgestaltung der Vorrichtung kann technisch einfach erreicht werden, dass die Vorrichtung zum Einsatzort der Messung transportiert wird und dann eine Optimierung der Sensorparameter und/oder der Analysestrategie unter Originalbedingungen erfolgt. Insbesondere ist es denkbar, die Vorrichtung an vergleichbare Orte zu transportieren und dort einen Sensor und/oder eine Auswerteeinheit zu optimieren. Anschließend werden die optimierte Analysestrategie und/oder die optimierten Sensorparameter, beispielsweise auf einer Speicherkarte hinterlegt, zusammen mit der Auswerteeinheit und/oder dem Sensor an einen Kunden geschickt, bei dem die entsprechende Messumgebung vorhanden ist. Hierdurch kann effizient eine Optimierung der Sensoren und/oder der Auswerteeinheit erfolgen, ohne direkt vor Ort sein zu müssen.

In besonders bevorzugter Ausgestaltung ist die KI-Einheit dazu ausgebildet, einen Sensorparameter zu optimieren, um eine Steuereinheit der Vorrichtung zu optimieren. Ergänzend oder alternativ ist die KI-Einheit dazu ausgebildet, eine Analysestrategie zu optimieren, um eine Auswerteeinheit der Vorrichtung zu optimieren. Die KI-Einheit weist hierfür eine Schnittstelle zum Austauschen von Daten zwischen der KI-Einheit und einer Messvorrichtung auf. Hierdurch kann die erfindungsgemäße Lehre als Nachrüstlösung verwirklicht werden, wobei eine entsprechende KI-Einheit in eine bereits vorhandene Vorrichtung integriert werden kann, um so den Sensorparameter und/oder die Analysestrategie zu optimieren. Es versteht sich, dass die Steuereinheit für den Metalloxid-Sensor und/oder die Analyseeinheit entsprechend für eine externe Konfiguration und einen entsprechenden Datenaustausch konfiguriert sind.

Sensorparameter können beispielsweise eine Temperaturkurve, also einen zeitlichen Verlauf der Temperatur, eine einzelne Temperatur oder mehrere einzelne Temperaturen und/oder eine Dauer einer Aufheizphase bis zum Erreichen einer vorgegebenen Temperatur umfassen. Bei der Angabe einer Dauer einer Aufheizphase kann auch die zeitliche Temperaturänderung mit umfasst sein. Die Sensorparameter können auch weitere sensorspezifische Daten umfassen, beispielsweise auch einen Messbeginn oder ggf. eine Rückmeldung an eine Steuereinheit, die die Sensoren mit den Sensorparametern ansteuert. Des Weiteren können Einstellungsparameter oder Kalibrierdaten in den Sensorparametern umfasst sein.

In einer bevorzugten Ausführungsform ist der Sensor vor Beginn einer Messung auf eine vorbestimmte Arbeitstemperatur aufgeheizt. Diese Arbeitstemperatur kann bevorzugt sensor- und/oder stoffspezifisch bzw. gasspezifisch sein für den zu vermessenden Stoff bzw. das zu vermessende Gas in der Probe oder in der zu messenden Flüssigkeit. Die Arbeitstemperatur kann beispielsweise bevorzugt auch spezifisch sein für die Klassifikation von mindestens zwei unterschiedlichen Proben oder unterschiedlichen Stoffen innerhalb einer Probe.

In einer bevorzugten Ausführungsform beruht die KI-Einheit zur Anpassung der Sensorparameter auf einem Lernalgorithmus. Der Lernalgorithmus kann beispielsweise evolutionäres Lernen und/oder bevorzugt bestärkendes Lernen verwenden, sodass aus bisher aufgenommenen Sensordaten neue Sensordaten zur Anpassung des Sensors oder der Sensoren mittels der KI-Einheit generiert werden können.

In einer bevorzugten Ausführungsform ist die KI-Einheit dazu ausgebildet, Rückschlüsse in Abhängigkeit von unterschiedlichen Parametern zu ziehen. Bevorzugt können Rückschlüsse in Abhängigkeit zwischen der Temperatursteuerkurve, dem Sensortyp, der Heiztemperatur, der Arbeitstemperatur, der zu messenden Probe, den zu vermessenden Stoffen innerhalb der Probe, deren Quantität und/oder Qualität gezogen werden. Optional kann auch zusätzlich oder alternativ zu einem der oben genannten Parameter die Temperatur der Heizplatte berücksichtigt werden. Auf diese Weise ist es möglich, eine schnellere Optimierung des Metalloxid-Sensors zu ermöglichen. Auch ist eine schnelle Optimierung für andere oder ähnliche Sensoren, bevorzugt andere Metalloxid-Sensoren, erfolgreich durchführbar.

Eine bevorzugte Vorrichtung umfasst ein Gehäuse, in dem die Vorrichtung angeordnet ist. Das Gehäuse hat bevorzugt eine verschließbare Öffnung, um Messungen in einer definierten Umgebung auszuführen. Somit lässt sich die Vorrichtung von der Außenwelt und der Umgebung hermetisch abriegeln. Durch die verschließbare Öffnung können Proben eingebracht und Sensoren ausgetauscht werden. Auf diese Weise ist es möglich, Messungen unter definierten Rahmenbedingungen durchzuführen, sodass die Messungen auch unabhängig von der Umgebung erfolgen können.

In einer vorteilhaften Ausgestaltung ist die Vorrichtung mobil und lässt sich einfach an Einsatzorte und Anwendungsumgebungen transportieren. So können die Sensoren bevorzugt vor Ort für eine Anwendung in der Anwendungsumgebung trainiert werden. Dies wird insbesondere durch ein Gehäuse mit einer Klappe oder Öffnung ermöglicht. Bevorzugt werden KI-Modelle für die optimalen Sensorparameter, z. B. Temperaturprofile, trainiert, die bevorzugt die Einflüsse der Einsatzumgebung widerspiegeln. In einer bevorzugten Ausführungsform können mehrere untrainierte Sensoren mit einem einfachen "Klick-Board" in der Vorrichtung, dem sogenannten Sensortrainer oder Trainer, "geklickt" und in der Anwendungsumgebung für die jeweilige Anwendung trainiert werden. Anschließend können diese trainierten Sensoren mit der entsprechenden gefundenen Sensorparameterkonfiguration in die tatsächliche Anwendung (vor Ort) verbaut werden. Das Training geschieht dabei bevorzugt vollautonom und/oder automatisiert. Die verwendeten Proben sind bekannt. Eine Untersuchung der Zusammensetzung erfolgt nicht. Vielmehr wird nur die Unterscheidung zwischen einzelnen Proben und die Wahl der Parameter trainiert.

In einer bevorzugten Ausführungsform erfolgt vor der Messung einer Probe eine Referenzmessung. Diese wird bevorzugt mit Luft, Reinluft, Umgebungsluft oder mit einer Referenzprobe durchgeführt. Die verwendete Luft oder Reinluft ist in diesen Fällen bekannt. Auch kann die Umgebungsluft beispielsweise mit einem anderen Sensor gemessen werden, sodass die darin enthaltenen Komponenten und Werte bekannt sind. Beispielsweise könnte Luft eingeströmt werden, die der Luft am geplanten Einsatzort des oder der Sensoren entspricht.

In einer bevorzugten Ausführungsform umfasst die Vorrichtung eine Speichereinheit zum Abspeichern von Daten. Besonders bevorzugt werden in der Speichereinheit Messdaten, Sensorparameter, optimierte Sensorparameter und/oder zugehörige Temperatursteuerkurven abgespeichert. Des Weiteren können ebenso bevorzugt Aufheizdauern für den Sensor und/oder für die Heizplatten der Probengefäße, Arbeitstemperaturen der Sensoren und/oder Trainings- oder Lerndaten abgespeichert werden. Weitere Daten bezüglich der Steuerung oder Automatisierung der Vorrichtung können ebenfalls gespeichert werden.

In einer weiter bevorzugten Ausführungsform ist die Steuereinheit dazu eingerichtet und ausgebildet, die Bewegungseinheit zu steuern. Auf diese Weise kann der Sensorkopf mit den Sensoren und/oder das Probengefäß in eine Messposition gebracht werden. Auch ist die Steuereinheit dazu eingerichtet, das Heizen der Heizplatte und/oder der Probe zu steuern. Ebenfalls werden bevorzugt durch die Steuereinheit zusätzlich alternativ die Sensoren im Sensorkopf mit Sensorparametern gesteuert. Hierbei können einzelne Sensoren oder der gesamte Sensorkopf gesteuert werden.

Durch die Steuereinheit kann die Bewegungseinheit mittels Aktoren ein Bewegen des Sensorkopfs, beispielsweise an einer Traverse, initiieren und ermöglichen, sodass der Sensorkopf über stationären Probengefäßen positioniert wird, bevor eine Messung erfolgt.

Alternativ können auch die Probengefäße unterhalb des Sensorkopfs bewegt werden. Auch in diesem Fall wird für das jeweilige Probengefäß eine Messposition angesteuert, sodass eine Messung der Proben in dem Probengefäß erfolgen kann.

In einer bevorzugten Ausführungsform sind die Probengefäße so ausgebildet, dass sie nach oben hin verschlossen sind, beispielsweise mittels eines durchstoßbaren oder öffenbaren Deckels. Das Probengefäß kann bevorzugt geöffnet werden, sobald der Sensorkopf in der Messposition relativ zum Probengefäß ist. Hierbei ist es möglich, dass der Sensorkopf teilweise in das Probengefäß hineinragen kann, wenn ein oberer Verschlussdeckel des Probengefäßes entfernt ist.

Alternativ ist es möglich, dass ein oberer Verschlussdeckel des Probengefäßes eine variable Öffnung beispielsweise in Form einer Art Ventil hat, sodass der Sensorkopf dieses automatisch öffnen und durch die vorzugsweise sich an den Probenkopf anschmiegende Öffnung der Deckplatte des Probengefäßes wenigstens teilweise hindurchbewegt werden kann, sodass die Stoffe innerhalb des Probengefäßes gemessen werden können. Auf diese Weise erfolgt eine möglichst unverfälschte Messung.

Die Erfindung betrifft auch Verfahren zum automatisierten Durchführen von Messungen und Sammeln von Messdaten sowie zum Erzeugen optimaler Sensorparameter eines Metalloxid-Sensors und/oder einer optimierten Analysestrategie einer Auswerteeinheit zur Bestimmung eines in einer Probe enthaltenen Stoffs oder zur Bestimmung von Trainingsdaten eines derartigen Metalloxid-Sensors und/oder einer derartigen Auswerteeinheit. Das Verfahren kann insbesondere eingesetzt werden, wenn die Güte von Lebensmitteln überprüft werden soll und Sensorparameter für eine solche Qualitätskontrolle von Lebensmitteln ermittelt werden sollen.

Das Verfahren kann mehrere Schritte umfassen, die auch mehrfach, teilweise mehrfach, gruppiert oder einzeln ausgeführt werden können.

Gemäß einem Schritt wird eine Messposition angefahren, in der ein Sensorkopf mit wenigstens einem Metalloxid-Gassensor oberhalb des Probengefäßes angeordnet ist. Das Anfahren der Messposition kann durch ein Bewegen des Sensorkopfs mit dem Gassensor und/oder durch ein Bewegen eines Probengefäßes für die zu ermittelnde und zu vermessende Probe erfolgen. Selbstverständlich können auch Sensorkopf und Probengefäß relativ zueinander bewegt werden.

In einem weiteren Schritt wird der Metalloxid-Gassensor mit einem vorbestimmten Sensorparameter oder mit mehreren Sensorparametern angesteuert. Die Sensorparameter können Temperaturkurven oder einzelne Temperaturwerte umfassen, beispielsweise bevorzugt Temperaturen zwischen 200 °C und 400 °C.

Ein weiterer Schritt sieht das Messen eines Stoffs vor, der in der Probe in dem Probengefäß umfasst ist. Die Messdaten, die von dem Sensor gemessen wurden, werden in einem weiteren Schritt ausgewertet, was bevorzugt in einer Auswerteeinheit erfolgen kann.

In einem weiteren Schritt werden Rückschlüsse gezogen zwischen den Messdaten und den Sensorparametern und/oder dem Stoff der zu vermessenden Probe, in der detektiert werden soll. Hier können auch die Messwerte mit Sensorparametern in Beziehung gesetzt, gemeinsam analysiert oder ausgewertet und weiterverarbeitet werden.

Ein weiterer Schritt sieht das Optimieren einer Anpassung der Sensorparameter basierend auf den Messdaten vor, um eine schnellere Anpassung des Sensors oder anderer, ähnlicher, beispielsweise baugleicher oder typgleicher Sensoren zu ermöglichen. Die Anpassung und Optimierung der Sensorparameter erfolgt während des Messbetriebs bzw. während des Sammelbetriebs und basiert auf einer KI-Einheit, die in die Lage versetzt wird, aus der Anpassung zu lernen und Trainingsdaten für andere Sensoren zu erstellen. Durch die Optimierung der Anpassung der Sensorparameter und den Einsatz der KI-Einheit wird eine höhere Genauigkeit der Klassifikationen unterschiedlicher, mindestens zweier verschiedener Proben erreicht. Auch erfolgt eine höhere Genauigkeit der Optimierung der Sensordaten.

Das oben beschriebene Verfahren kann beispielsweise in einem Programmcode implementiert sein, der das Durchführen des Verfahrens initiiert, wenn der Programmcode auf einem Computer ausgeführt wird. Dieser Programmcode kann auf einem Computerprogrammprodukt, beispielsweise einer Festplatte oder einem anderen Speichermedium, gespeichert sein.

Der Sensorkopf trägt die Sensoren in dem Sinne, dass die Sensoren am oder im Sensorkopf angeordnet sind. Die Sensoren können beispielsweise an einer Außenwand des Sensorkopfs, vorzugsweise lösbar, angeordnet sein. Sie können angeklemmt oder aufgesteckt sein. Der Sensorkopf kann mehrere Sensoren tragen. Während der Messung ist der Sensorkopf oberhalb der Probengefäße angeordnet, wobei der Sensorkopf teilweise in die oben offenen Probengefäße hineinragen kann.

Im Rahmen der Optimierung kann ein Wert berechnet werden, der eine Trennbarkeit der einzelnen Proben beschreibt. Dieser Wert gibt an, wie gut die Sensoren bzw. die Vorrichtung in der Lage ist, die verschiedenen Proben zu unterscheiden. Eine Optimierung erfolgt insbesondere im Sinne einer Anpassung der Sensorparameter mit dem Ziel einer besseren Trennbarkeit und wird abgebildet durch einen Score, der einen Abstand zwischen verschiedenen Klassen und die Streuung innerhalb der Klassen einbezieht.

Ein F1-Score kann ein Trennbarkeitswert oder Fitnesswert sein. Eine Fitnessfunktion des Optimierungsalgorithmus maximiert die Trennbarkeit der Proben, vergrößert den Abstand zwischen den Klassen und minimiert die Streuung innerhalb der Klassen. Beispielsweise werden die Heizkurvenparameter, wie die Oberflächentemperaturen und deren Abfolge, entsprechend angepasst. Die Fitness kann anhand der Trennbarkeit der Proben bewertet werden.

Der Ausdruck Sensorparameter ist vorliegend breit zu verstehen und kann insbesondere mehrere Parameter zur Steuerung der hier offenbarten Vorrichtung umfassen. Insbesondere kann als Sensorparameter eine Heizkurve eines Metalloxid-Sensors, eine Betriebsspannung, eine Messzeit, eine Heizkurve für ein Probenbehältnis, eine Öffnungsstellung oder ein Öffnungsgrad der Öffnung, eine Ausrichtung des Sensorkopfs etc. verstanden werden.

Unter Analysestrategie ist insbesondere eine Auswahl und/oder Reihenfolge verschiedener Analyseschritte, Algorithmen und Verfahren zu verstehen. Ferner kann die Analysestrategie verschiedene Verfahren zur Datenaufbereitung, Filterung und/oder Rauschunterdrückung umfassen.

Im Folgenden werden kursorisch mögliche Anwendungen der hier offenbarten Erfindung skizziert.

Ein Versuchsaufbau kann beispielsweise Proben in 3 Gläsern mit einem Gehalt von jeweils 0 g, 10 g und 20 g Essigsäure pro Liter umfassen. Die Gläser werden zu einem Drittel gefüllt, bei einer maximalen Füllmenge von 100 ml enthalten die Gläser ca. 33 ml.

Die Sensorparameter sollen so angepasst werden, dass das KI-Modell möglichst genaue Vorhersagen über den Essigsäuregehalt in unbekannten Wasser-Essig-Mischungen treffen kann, um so eine optimale Analysestrategie und/oder optimale Sensorparameter zu bestimmen. Dies wird durch iterative Anpassung der Parameter durch die KI-Einheit erreicht.

Alternativ können auch Feststoffe in den sogenannten Sensortrainer gefüllt werden. Beispielsweise können 3 Gläser Kunststoffproben der gleichen Kunststoffgruppe in unterschiedlicher Qualität enthalten sein, wobei die Kunststoffproben wie folgt kategorisiert sein können: gut, noch verwendbar, nicht verwendbar.

Ziel ist die Anpassung der Sensorparameter, sodass die Auswerteeinheit in der Lage ist, eine gute Zuordnung von Kunststoffproben unbekannter Qualität zu treffen. Dies wird durch iterative Anpassung der Parameter durch die KI-Einheit erreicht. Ferner kann die KI-Einheit eine dafür passende Analysestrategie bestimmen.

Die Proben werden mit einem Deckel verschlossen und auf die gewünschte Temperatur temperiert. Nach dem Erreichen einer Zieltemperatur kann die Optimierung erfolgen.

Die Sensorparameter können in diesem Beispiel eine Aufheizkurve der MOX-Sensoren beschreiben. Die Kurve kann aus beliebig vielen Temperaturpunkten zwischen beispielsweise 50 °C und 400 °C bestehen, die in beliebiger Reihenfolge und mit beliebiger Dauer miteinander kombiniert werden können.

Es versteht sich, dass die Sensorparameter um weitere Größen wie z. B. definierte Abkühlphasen erweitert werden können.

Der Ablauf der Parameteroptimierung beginnt vorzugsweise mit der Bestimmung der initialen Parameter, die entweder zufällig oder basierend auf dem Wissen der KI-Einheit festgelegt werden. Anschließend werden alle oder nur einzelne Proben mit verschiedenen Heizprofilen gemessen. Die Proben können Feststoffe, Gase oder Flüssigkeiten sein. Die KI-Einheit wertet dann die Messdaten vorzugsweise aller Proben aus und bestimmt eine Fitness der verschiedenen Heizprofile oder Parameter. Danach wird überprüft, ob bestimmte Abbruchkriterien erfüllt sind.

Basierend auf den Ergebnissen des Fitnesstests werden neue Parameter selektiert, angepasst und generiert, was durch einen Optimierungsalgorithmus erfolgt. Dieser Prozess wird iterativ wiederholt, bis die besten Sensorparameter gefunden sind, d. h. ein Abbruchkriterium, wie Mindestfitness oder Maximalanzahl von Durchgängen, erreicht wird.

Die Bestimmung der initialen Parameter umfasst mehrere Aspekte. Zunächst kann ein Temperaturprofil erstellt werden, das beispielsweise aus 10 spezifischen Temperaturpunkten besteht. Diese Punkte definieren die Temperaturen, die während des Messprozesses erreicht und gehalten werden sollen. Zusätzlich zu den Temperaturpunkten können weitere Parameter festgelegt werden, die die Ansteuerung der Sensoren betreffen. Diese können beispielsweise die Messdauer pro Glas, also Probengefäß, die Haltezeit bei bestimmten Temperaturen und die Abkühlphase umfassen.

Darüber hinaus gibt es anpassbare Parameter, wie die Temperatur der Gläser, in denen die Proben gehalten werden. Diese Temperatur kann angepasst werden, um die Messbedingungen zu optimieren. Ebenso kann die Größe des Einflusses der Umgebungsluft auf die Messungen berücksichtigt und angepasst werden.

Diese initialen Parameter werden entweder zufällig oder basierend auf dem Wissen der KI-Einheit aus Messungen mit ähnlichen Proben festgelegt.

Anschließend werden vorzugsweise alle Proben, die sich in den Gläsern befinden, mittels verschiedener Heizprofile gemessen. Die Messungen dienen dazu, Daten zu sammeln, die später von der KI-Einheit ausgewertet werden, um die Fitness der verschiedenen Heizprofile zu bestimmen.

Eine Datenaufnahme für ein Glas erfolgt vorzugweise anhand der folgenden Schritte: Messkopf über Reinigungsstation platzieren; Sensoren mit Frisch-/Nulluft ca. 15 Minuten spülen; Messkopf über dem Glas mit Probe platzieren; Messdaten aufnehmen. Die Dauer der Aufnahme erfolgt je nach Heizprofil, dabei kann das Heizprofil kontinuierlich wiederholt werden. Die Messdauer beträgt beispielsweise 5 bis 15 Minuten. Die Gesamtzeit für drei Gläser beträgt dabei 1 bis 1,5 Stunden. Die Messdaten werden in einer Speichereinheit abgelegt.

Kenngrößen der Eingangswerte sind die Messdaten an vorzugsweise definierten Messpunkten des verwendeten Heizprofils. Diese Merkmale können um weitere Größen wie Glastemperatur, Luftdruck, Luftfeuchtigkeit, Lufttemperatur, Tageszeit etc. erweitert werden.

Fehlende Werte können behandelt werden, indem Methoden wie Mittelwert- oder Median-Imputation angewendet werden. Alternativ kann auch die K-Nearest Neighbors (KNN)-Imputation genutzt werden. Ausreißer können durch die Z-Score- oder IQR-Methode (Interquartilabstand) erkannt und behandelt werden.

Eine Skalierung der Daten kann durch Min-Max-Skalierung erfolgen. Alternativ kann auch eine Standardisierung mittels Z-Score-Normalisierung verwendet werden. Eine Kodierung kategorialer Variablen kann durch One-Hot-Encoding oder Label-Encoding erfolgen. Ferner kann eine Korrektur von Luftdruck, Luftfeuchtigkeit, Lufttemperatur und Tageszeit erfolgen. Bekannte Messgrößen können ebenfalls berücksichtigt werden. Der Einfluss der Luftfeuchtigkeit auf Messwerte kann zum Beispiel korrigiert werden.

Die Daten werden vorzugsweise in Trainings- und Testmenge geteilt. Aus der Trainingsmenge kann mittels KI ein Sensorparameter und/oder eine Analysestrategie ermittelt werden. Die Testmenge kann genutzt werden, um die Fitness der Konfigurationen, also insbesondere des Sensorparameters und/oder der Analysestrategie, zu bestimmen.

Zur Dimensionalitätsreduktion stehen verschiedene Verfahren zur Verfügung. Die Principal Component Analysis (PCA) reduziert die Anzahl der Variablen, indem sie die wichtigsten Komponenten extrahiert, die die meiste Varianz erklären. Alternativ kann die Linear Discriminant Analysis (LDA) verwendet werden, die die Dimensionen reduziert, indem sie die Klassenunterschiede maximiert und die Varianz innerhalb der Klassen minimiert. Zusätzlich kann Feature Engineering durchgeführt werden, wobei durch Korrelationsanalyse oder Lasso-Regression Features ausgewählt und durch Polynomial Features oder Interaktionsfeatures neue Features erstellt werden.

Für die Auswertung können verschiedene Methoden eingesetzt werden. Die KI-Einheit kann in der Lage sein, verschiedene Modelle und Methoden zu testen und die beste Bewertungsmethode bzw. Analysestrategie für den Anwendungsfall zu bestimmen.

Bei einem Regressionsproblem können eine lineare Regression oder eine multiple lineare Regression verwendet werden, die Beziehungen zwischen einer abhängigen und mehreren unabhängigen Variablen modelliert.

Alternativ können eine nichtlineare Regression und eine polynomiale Regression eingesetzt werden, um nichtlineare Beziehungen durch Polynomterme zu modellieren. Eine logistische Regression kann für binäre Klassifikationsprobleme verwendet werden, lässt sich aber auch für Regressionsprobleme anpassen. Eine regularisierte Regression umfasst Methoden wie Ridge Regression, die einen L2-Strafterm hinzufügt, um Überanpassung zu vermeiden, und eine Lasso-Regression, die einen L1-Strafterm hinzufügt, um weniger wichtige Features zu eliminieren.

Baumbasierte Methoden umfassen Entscheidungsbäume, die einfach zu interpretieren sind, aber anfällig für Überanpassung sind, und Random Forests, ein Ensemble von Entscheidungsbäumen zur Verbesserung der Genauigkeit und Robustheit. Support Vector Regression (SVR) kann sowohl für lineare Beziehungen (lineare SVR) als auch für nichtlineare Beziehungen (nichtlineare SVR mit Kernel-Tricks wie dem Radial Basis Function (RBF) Kernel) verwendet werden. Schließlich können neuronale Netze eingesetzt werden, wobei einfache neuronale Netze für einfache Regressionsprobleme und tiefe neuronale Netze für komplexere und größere Datensätze geeignet sind.

Eine lineare Diskriminanzanalyse (LDA) findet eine lineare Kombination von Merkmalen, die die Klassen am besten trennt. Support Vector Machines (SVM) sind ebenfalls eine gängige Methode zur Klassifikation. Alternativ können logistische Regression, die die Wahrscheinlichkeit einer binären oder mehrklassigen Zielvariable modelliert, Entscheidungsbäume, die einfach zu interpretieren, aber anfällig für Überanpassung sind, und Random Forests, ein Ensemble von Entscheidungsbäumen zur Verbesserung der Genauigkeit und Robustheit, verwendet werden. Gradient Boosting Machines (GBM) bauen sequentiell Bäume, wobei jeder Baum die Fehler des vorherigen korrigiert.

Die KNN-Klassifikation klassifiziert Datenpunkte basierend auf den Klassen der nächsten Nachbarn. Neuronale Netze können ebenfalls eingesetzt werden, wobei einfache neuronale Netze für einfache Klassifikationsprobleme und tiefe neuronale Netze für komplexere und größere Datensätze geeignet sind.

Mit der Testmenge wird die Eignung für jede Sensorkonfiguration bestimmt. Ziel ist es, eine Vergleichbarkeit der verschiedenen Parameter zu erreichen, um daraus Schlussfolgerungen ziehen zu können. Für jede Sensorkonfiguration wird ein Score berechnet.

Eine Score Regression bezieht sich auf die Bewertung und Quantifizierung der Qualität von Vorhersagen in Regressionsmodellen. Der Mean Squared Error (MSE) misst den mittleren quadratischen Fehler zwischen den vorhergesagten und den tatsächlichen Werten. Der MSE wird berechnet, indem die Differenzen zwischen den vorhergesagten und den tatsächlichen Werten quadriert werden und dann der Durchschnitt dieser quadrierten Differenzen gebildet wird. Er ist ein Maß für die Güte eines Schätzers. Er kann verwendet werden, um verschiedene Modelle, in diesem Fall die verschiedenen Sensorkonfigurationen, zu vergleichen und das Modell mit dem kleinsten MSE als das beste Modell auszuwählen.

Ein F1-Score ist vorzugsweise eine Metrik, die zur Bewertung der Genauigkeit eines Klassifikationsmodells verwendet werden kann. Er kombiniert Präzision und Recall - Rückruf - in einer einzigen Zahl und kann wie folgt berechnet werden:
Eine Präzision (Precision) beschreibt den Anteil der positiven Vorhersagen, die korrekt klassifiziert wurden, im Vergleich zur Gesamtzahl der positiven Fälle im Datensatz. Die Präzision ergibt sich aus True Positive /(True Positive + False Positive), also korrekt positiven Vorhersagen geteilt durch die Summe der korrekt positiven Vorhersagen und falsch positiven Vorhersagen. Der Recall - Rückruf - beschreibt den Anteil der tatsächlich positiven Beispiele, die korrekt als positiv erkannt wurden und kann wie folgt berechnet werden: Recall=True Positives / (True Positives + False Negatives), also korrekt positive Vorhersagen geteilt durch die Summe der korrekt positiven Vorhersagen und falsch negativen Vorhersagen. Der F1-Score bildet das harmonische Mittel von Präzision und Recall und berechnet sich wie folgt: F1 =( 2· Präzision · Rückruf) / (Präzision + Rückruf).

Der F1-Score liegt zwischen 0 und 1, wobei 1 die beste mögliche Leistung darstellt. Ein vordefiniertes Abbruchkriterium im Rahmen dieser Offenbarung könnte sein, dass ein F1-Score von mehr als 0,9 erreicht wird. In anderen Worten beschreibt Präzision (Precision) den Anteil der korrekt vorhergesagten positiven Beispiele, also Messdaten bzw. Messkurven, die eine korrekte Messung der Stoffe in der Probe ermöglichen, an allen vorhergesagten positiven Beispielen, also an allen aufgenommenen Messkurven. Recall (Sensitivität) hingegen misst den Anteil der korrekt vorhergesagten positiven Beispiele, also korrekten Messkurven, an allen tatsächlichen positiven Beispielen, also allen Messkurven, die theoretisch möglich wären.

Der Mean Squared Error (MSE) ist eine gängige Metrik zur Bewertung der Genauigkeit eines Regressionsmodells und beschreibt den durchschnittlichen quadratischen Fehler zwischen den vorhergesagten Werten und den tatsächlichen Werten. Er kann wie folgt berechnet werden: MSE = (1/n) * Σ(tatsächlich - vorhergesagt)² , wobei n die Anzahl der Beobachtungen ist. Ein niedriger MSE-Wert bedeutet, dass die vorhergesagten Werte nahe an den tatsächlichen Werten liegen, was auf ein gutes Modell hinweist. Ein hoher MSE-Wert deutet darauf hin, dass das Modell größere Fehler bei den Vorhersagen macht.

Zusätzlich zu dem F1-Score kann ein Algorithmus verwendet werden, der den Abstand der Mittelwerte aller Klassen mit der Varianz in Beziehung setzt. In einem ersten Schritt wird für jede Klasse/Probe die Konfidenzellipse berechnet, die 75 % der Datenpunkte der Klasse enthält. Die Konfidenzellipse hilft, die Verteilung und Streuung der Datenpunkte zu visualisieren und zu verstehen, wie gut die Datenpunkte innerhalb eines Clusters gruppiert sind. Im zweiten Schritt werden die minimalen Abstände aller Konfidenzellipsen berechnet und addiert. Ein großer Wert weist auf eine gute Trennbarkeit hin, ein kleiner Wert auf eine schlechte Trennbarkeit; bei negativen Werten ist keine eindeutige Trennbarkeit möglich.

Referenzmessungen und -werte spielen eine entscheidende Rolle im Optimierungsprozess. Sie dienen als Basis zur Bewertung der Parametereffizienz und -leistung. Durch den Vergleich der Modellvorhersagen mit den bekannten Referenzwerten können Metriken wie der Mean Squared Error (MSE) für Regressionsprobleme und der F1-Score für Klassifikationsprobleme berechnet werden. Diese Metriken ermöglichen eine objektive Bewertung und Optimierung der Sensorkonfigurationen.

Die Vorhersagegüte beschreibt die Genauigkeit und Zuverlässigkeit der Vorhersagen, also der ermittelten Sensorparameter und/oder der ermittelten Analysestrategie, die durch das KI-Modell unter bestimmten Bedingungen erzielt werden. Sie wird durch Metriken wie den Mean Squared Error (MSE) für Regressionsprobleme und den F1-Score für Klassifikationsprobleme quantifiziert. Eine hohe Vorhersagegüte zeigt an, dass das Modell präzise und verlässliche Vorhersagen trifft.

Um sicherzustellen, dass der Optimierungsprozess effizient und zielgerichtet abläuft, wird bei jeder Iteration geprüft, ob ein Abbruchkriterium erreicht wurde. Dabei werden zwei Hauptkriterien überprüft. Erstens wird der Prozess gestoppt, sobald ein vordefinierter Fitnesswert erreicht wird. Dieser Wert dient als Indikator dafür, dass die Lösung gut genug ist und keine weiteren Iterationen notwendig sind. Zweitens wird der Prozess auch nach einer bestimmten Anzahl von Iterationen beendet, unabhängig davon, ob der gewünschte Fitnesswert erreicht wurde oder nicht.

Angenommen, die Sensorparameter werden so angepasst, dass die Auswerteeinheit mittels der durch die KI-Einheit bestimmten Sensorparameter und/oder Analysestrategie einen Essigsäuregehalt in unbekannten Wasser-Essig-Mischungen vorhersagen kann. Ein MSE von beispielsweise 0,05 würde eine hohe Vorhersagegüte beschreiben.

Ein genetischer Algorithmus zur Optimierung der Parameter von MOX-Sensoren kann mit mindestens vier Basisheizprofilen beginnen, die die Ausgangspopulation und somit die erste Generation der Eltern bilden. In der ersten Iteration wird eine vollständige Messung mit Datenauswertung durchgeführt. Anschließend wird die Population nach dem Score sortiert. Aus den vier besten Eltern werden neue Kinder erzeugt. Das Generieren der Kinder erfolgt durch Crossover und Mutation der Elternprofile. Dabei werden die Temperaturwerte der Elternteile kombiniert, und ein zufälliges Temperaturelement wird geändert, um die Vielfalt der Population zu erhöhen und die Wahrscheinlichkeit zu verringern, dass der Algorithmus in einem lokalen Minimum stecken bleibt. Anschließend erfolgt wieder eine vollständige Messung mit Datenauswertung. Die Schritte werden so lange wiederholt, bis ein Abbruchkriterium erfüllt ist. Eine Optimierung mit 10 Iterationen und 3 Proben könnte also ca. 1000 Minuten bzw. 17 Stunden in Anspruch nehmen.

Es ist denkbar, dass die KI auch die Heizplatte unter der Probe steuert, um die Temperaturbedingungen zu optimieren. Dies könnte durch die Integration zusätzlicher Steuerparameter in den Optimierungsalgorithmus erfolgen. Die Temperatur der Heizplatte könnte in Abhängigkeit von den gewünschten Messbedingungen und den spezifischen Anforderungen der Probensteuerung angepasst werden. Die Steuerung der Heizplatte könnte die Genauigkeit und Zuverlässigkeit der Messungen beeinflussen, insbesondere wenn die Temperatur der Probe eine kritische Rolle spielt.

Die Vorhersagegüte ist stark abhängig von der Temperatur, der Ansteuerung der Heizplatte und der Temperatur der Probe. Die Sensorparameter, einschließlich der Aufheizkurve der MOX-Sensoren, beeinflussen die Messgenauigkeit und damit die Vorhersagegüte. Die Temperatur der Probe, die durch die Heizplatte geregelt wird, spielt eine entscheidende Rolle bei der Bestimmung der Messbedingungen. Um die Vorhersagegüte zu maximieren, müssen diese Faktoren sorgfältig optimiert und angepasst werden. Der genetische Algorithmus könnte diese Variablen berücksichtigen und die Heizprofile iterativ anpassen, um die besten Vorhersagen zu erzielen.

Die Erfindung wird nachfolgend anhand einiger ausgewählter Ausführungsbeispiele im Zusammenhang mit den beiliegenden Zeichnungen näher beschrieben und erläutert. Es zeigen:
- Figur 1: eine Prinzipskizze einer Ausführungsform der vorliegenden Erfindung;
- Figur 2: eine schematische Darstellung der Vorrichtung;
- Figur 3: eine weitere schematische Darstellung der Vorrichtung aus Figur 2;
- Figur 4: eine schematische Darstellung einer alternativen Ausführungsform; und
- Figur 5: eine schematische Darstellung des Verfahrensablaufs.

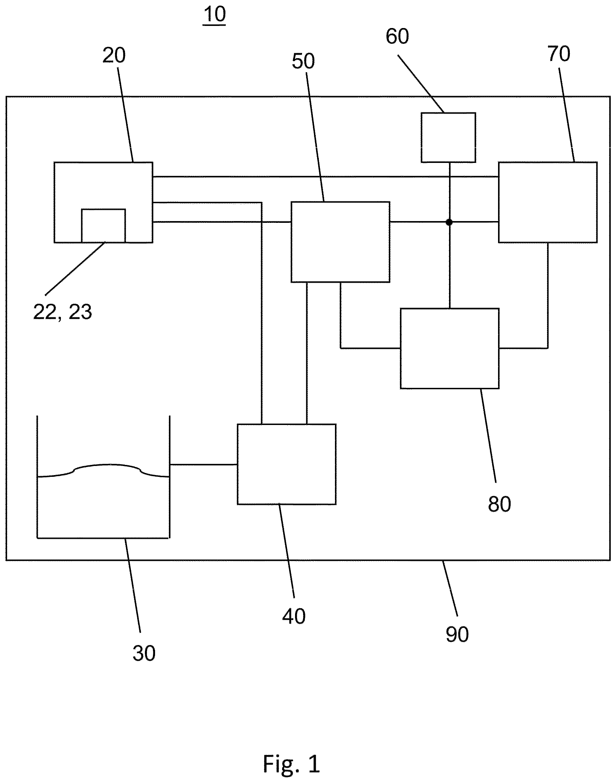

In den Figuren 1 bis 4 sind verschiedene Ausführungsformen der erfindungsgemäßen Vorrichtung 10 dargestellt.

Figur 1 zeigt die Vorrichtung 10 umfassend einen Sensorkopf 20 mit einem oder mehreren Sensoren 22, von denen hier ein Sensor 22 dargestellt ist. Der Sensor 22 ist beispielsweise ein Metalloxid-Sensor 23.

Die Vorrichtung 10 umfasst weiter exemplarisch ein Probengefäß 30 zur Aufnahme einer zu vermessenden Probe, die beispielsweise eine Flüssigkeit, ein Feststoff oder ein Gas sein kann.

Eine Bewegungseinheit 40 dient zum Bewegen der oder des Probengefäßes 30 und/oder des Sensorkopfs 20 in eine Messposition, in der der Sensorkopf 20 oberhalb oder teilweise innerhalb eines der Probengefäße 30 angeordnet ist.

Eine Steuereinheit 50 ist dazu ausgebildet, den Sensor 22 oder Sensorkopf 20 anzusteuern. Dazu werden Sensorparameter an den oder die Sensoren 22 übermittelt. Die Sensorparameter können beispielsweise in einer optionalen Speichereinheit 60 abgespeichert sein, die in der Steuereinheit 50 integriert sein kann.

Die Vorrichtung 10 umfasst weiter eine Auswerteeinheit 70, die von dem Sensorkopf 20 bzw. den enthaltenen Sensoren 22 Messdaten empfängt und diese Messdaten auswertet. Die Auswerteeinheit 70 kann eine KI-Einheit umfassen, die dazu ausgebildet ist, Rückschlüsse zwischen den Messdaten und den Sensorparametern, mit denen der Sensor 22 angesteuert wird, zu ziehen. Weiter dient die Auswerteeinheit 70 dazu, eine Anpassung der Sensorparameter basierend auf den Messdaten zu optimieren. Sie ermöglicht eine schnellere Anpassung des Sensors 22 oder anderer, beispielsweise baugleicher Sensoren 22. Auch ist eine Optimierung der Anpassung schnell möglich.

Es versteht sich, dass die KI-Einheit 80 auch als eigenständige Einheit mit entsprechenden Schnittstellen ausgebildet sein kann.

Die KI-Einheit 80 ist dazu eingerichtet, dass die Anpassung und die Optimierung der Sensorparameter für die Steuereinheit 50 während des Messbetriebs erfolgt, also während eine Messung einer Probe in dem Probengefäß 30 durch den Sensorkopf 20 bzw. einen Sensor 22 erfolgt.

Mittels der KI-Einheit 80 wird die Anpassung und Optimierung der Sensorparameter durchgeführt. Dabei wird die KI-Einheit 80 in die Lage versetzt, aus der Anpassung zu lernen und Trainingsdaten für Sensoren 22 zu erstellen. Diese Trainingsdaten können ebenfalls in der Speichereinheit 60 abgespeichert werden. Die von der KI-Einheit 80 erzeugten Trainingsdaten können Sensorparameter umfassen, beispielsweise Temperaturkurven oder Temperaturzyklen oder auch stationäre Temperaturdaten. Diese können direkt an die Steuereinheit 50 geliefert werden. Alternativ ist es möglich, dass die Steuereinheit 50 initiiert wird, sich die entsprechenden Sensordaten aus der Speichereinheit 60 abzurufen.

In der hier gezeigten bevorzugten Ausführungsform ist die Vorrichtung 10 in einem Gehäuse 90 angeordnet. Das Gehäuse 90 ermöglicht eine hermetische Abriegelung von der Umgebung, sodass innerhalb des Gehäuses 90 ein eigenes "Biotop" gebildet wird, das von der außenliegenden Umwelt entkoppelt ist. Zu diesem Zweck ist das Gehäuse 90 bevorzugt komplett geschlossen und bietet eine luftdichte Abdichtung. Das Gehäuse 90 ermöglicht es also, Messungen in einer vorgegebenen und vordefinierten Umgebung stattfinden zu lassen.

In einer bevorzugten Ausführungsform kann das Gehäuse 90 eine verschließbare Öffnung aufweisen, sodass die einzelnen Probengefäße 30 einfach ausgetauscht oder neu befüllt werden können.

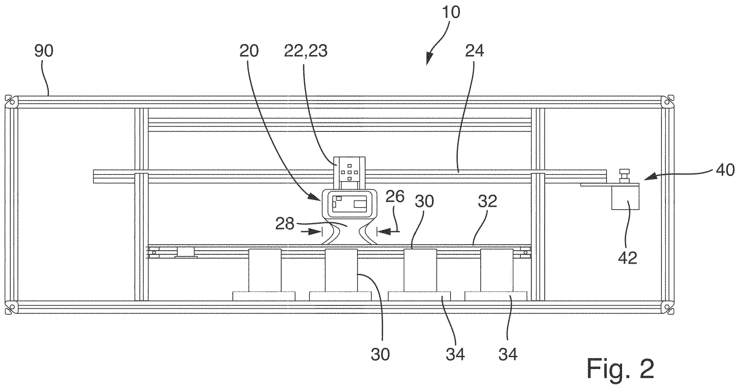

Figur 2 zeigt eine Prinzipskizze der Vorrichtung 10 mit einem Sensorkopf 20, der an einer Traverse 24 beweglich befestigt ist, sodass er in seiner Position verändert werden kann. Dies bietet die Möglichkeit, den Sensorkopf 20 in eine Messposition zu fahren, in der er oberhalb eines der Probengefäße 30 angeordnet ist, um eine Messung mit seinen Sensoren 22 bzw. Metalloxid-Sensoren 23 durchzuführen. Der Sensorkopf 20 umfasst mehrere Sensoren 22, bevorzugt Metalloxid-Sensoren 23. Eine Tunnelverbindung 26 schafft einen abgeschlossenen Messraum 28, der sich an das Probengefäß 30 in der Messposition anschließt und den Sensorkopf 20 mit den Sensoren 22 mitumfasst. Auf diese Weise werden von den Sensoren 22 lediglich das aus der Probe in dem Probengefäß 30 austretende Gas und die darin enthaltenen Stoffe gemessen. Die Tunnelverbindung 26 kann muffenartig oder manschettenartig ausgeführt sein und stellt eine Ummantelung zur unterbrechungsfreien Verbindung zwischen Sensorkopf 20 und Probengefäß 30 her.

Vorzugsweise können die Probengefäße 30 an ihrer Oberseite mit einer Gefäßabdeckung 32 mit einzelnen Öffnungen abgedeckt sein. Auf diese Weise lässt sich der Austritt von Stoffen aus den Proben sowie von Gasen steuern. Zudem wird sichergestellt, dass keine Luft von außen aus dem Gehäuse 90 in den Messraum 28 eintritt. Die Gefäßabdeckung 32 kann einstückig sein und sich über alle Probengefäße 30 erstrecken. Sie hat dann für jedes der einzelnen Probengefäße 30 eine entsprechende Öffnung. Diese Öffnung kann bevorzugt verschließbar sein. Dies kann etwa durch eine Art Diaphragma oder eine Manschette aus Kunststoff oder Gummi realisiert sein.

In der hier gezeigten Ausführungsform ist der Sensorkopf 20 an der Traverse 24 horizontal beweglich. Er kann zusätzlich eine vertikale Bewegung durchführen. Gesteuert wird die Bewegung des Sensorkopfs 20 durch die Bewegungseinheit 40, die in dem Gehäuse 90 oder außerhalb des Gehäuses 90 angeordnet sein kann. Als Aktor für die Bewegung dient ein Schrittmotor 42, der von der Bewegungseinheit 40 angesteuert wird, und eine nicht dargestellte Mechanik.

In der hier gezeigten Ausführungsform sind die Probengefäße 30 stationär angeordnet, also nicht beweglich. Jedes der einzelnen Probengefäße 30 ist auf einer Heizplatte 34 angeordnet, die separat angesteuert werden kann, vorzugsweise durch die (nicht gezeigte) Steuereinheit 50. Auf diese Weise kann jedes einzelne Probengefäß 30 individuell beheizt werden.

Das Gehäuse 90 kann, wie hier gezeigt, durch mehrere Profilstangen 92 aufgebaut sein, an denen transparente oder nichttransparente Seitenteile montiert sein können, die in der Figur nicht gezeigt sind.

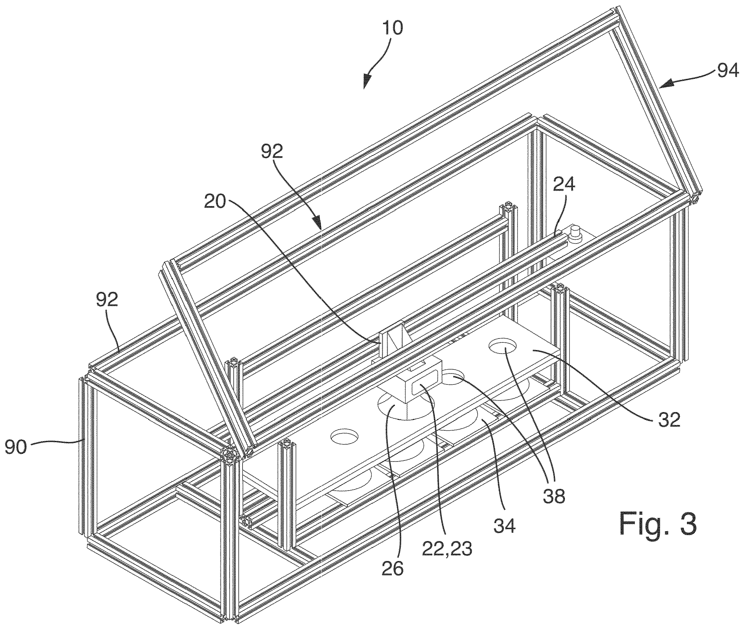

Figur 3 zeigt die Ausführungsform der Vorrichtung 10 mit einem Gehäuse 90, das ebenfalls aus Profilstangen 92 aufgebaut ist. Das Gehäuse 90 hat einen Deckel 94, der geöffnet werden kann, um Zugang zu der Vorrichtung 10 zu ermöglichen. In der hier gezeigten Ausführungsform sind die Probengefäße 30 stationär angeordnet, die auf Heizplatten 34 in Form von Heizfolien positioniert sind. Die Vorrichtung umfasst vier Probengefäße 30 mit den entsprechenden Heizplatten 34. Eine Gefäßabdeckung 32 schließt die Probengefäße 30 nach oben ab, wobei an der jeweiligen Position der Probengefäße 30 eine Öffnung 38 in der Gefäßabdeckung 32 vorgesehen ist.

Neben den vier Probengefäßen 30 ist eine weitere Messstelle angeordnet, die ebenfalls durch die Gefäßabdeckung 32 abgedeckt ist und eine entsprechende Öffnung 38 aufweist. Wird der bewegliche Sensorkopf 20 in diese sogenannte Referenzposition an der Traverse 24 gefahren, so kann eine Referenzmessung des Sensors 22 erfolgen. Hierbei wird die in dem Gehäuse 90 vorhandene Luft als Referenzprobe vermessen. Alternativ kann auch hier ein Probengefäß 30 in Form eines Referenzgefäßes vorgesehen sein, das beispielsweise mit Reinluft, Referenzluft oder Umgebungsluft gefüllt sein kann. Möglich ist auch, alternativ eine Messung bei geöffnetem Deckel 94 durchzuführen und so die Umgebungsluft außerhalb der Vorrichtung 10 als Referenz zu vermessen. Denkbar ist auch, dass eine spezielle Referenzprobe mit zu vermessenden Stoffen, die in ihrer Konzentration bekannt sind, verwendet wird.

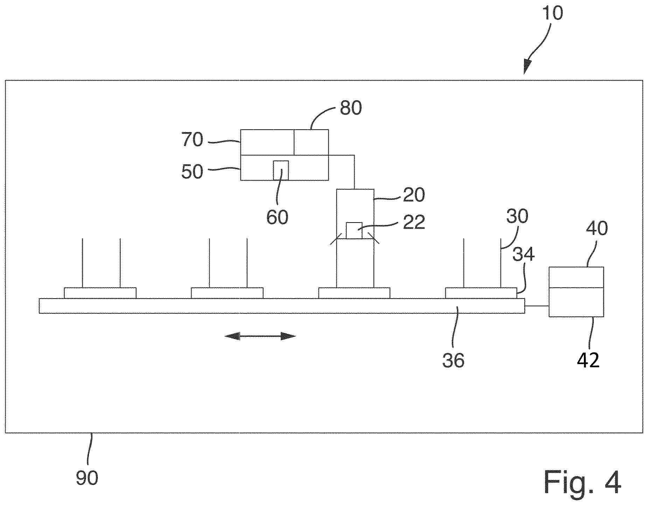

Figur 4 zeigt eine alternative Ausführungsform der Vorrichtung 10 in einem Gehäuse 90. In der hier gezeigten Form ist der Sensorkopf 20 mit den Sensoren 22 stationär angeordnet, während die Probengefäße 30 horizontal beweglich sind. Die Probengefäße 30 sind jeweils auf einer eigenen Heizplatte 34 oder Heizmatte angeordnet, die auf einem Schlitten 36 positioniert sind. Der Schlitten 36 wird über einen Schrittmotor 42 von der Bewegungseinheit 40 angesteuert und in seiner Position gemäß dem Pfeil 44 horizontal bewegt, sodass eines der Probengefäße 30 in einer Messposition unterhalb des Sensorkopfs 20 angeordnet ist. Zwischen dem Probengefäß 30, das in der Messposition ist, und dem Sensorkopf 20 wird ein Messraum 28 aufgespannt, der mittels einer Tunnelverbindung 26 zwischen Sensorkopf 20 und Probengefäß 30 gebildet wird.

In der hier dargestellten Ausführungsform sind die Steuereinheit 50, die Auswerteeinheit 70 und die KI-Einheit 80 ebenfalls in dem Gehäuse 90 angeordnet. Eine Speichereinheit 60 ist in die Steuereinheit 50 integriert und dient zur Speicherung von Sensorparametern, Messdaten der Auswerteeinheit 70 sowie anderen Konfigurationsdaten einschließlich Steuerdaten zur Steuerung der Probengefäße 30 in die vorgegebenen Messpositionen. Die Steuereinheit 50 kann bevorzugt dazu ausgebildet sein, mit der Bewegungseinheit 40 und der Auswerteeinheit 70 einen automatisierten Messablauf zum Sammeln von Sensorparametern der Sensoren 22 durchzuführen. Alternativ können diese Einheiten auch (teilweise) außerhalb des Gehäuses 90 angeordnet sein.

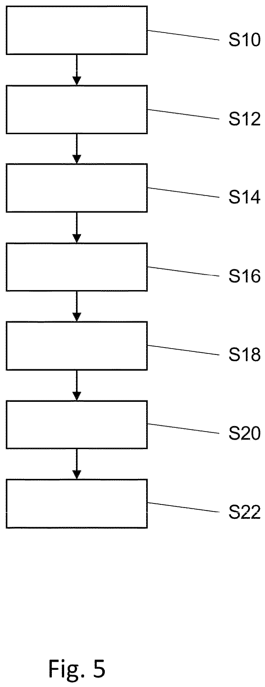

Figur 5 zeigt einen schematischen Ablauf eines Verfahrens zum automatisierten Durchführen von Messungen und Sammeln von Messdaten und zum Erzeugen von optimalen Sensorparametern eines Metalloxid-Sensors und/oder einer optimierten Analysestrategie einer Auswerteeinheit zur Bestimmung eines in einer Probe enthaltenen Stoffs oder zur Bestimmung von Trainingsdaten eines derartigen Metalloxid-Sensors und/oder einer derartigen Auswerteeinheit.

In einem ersten Schritt S10 erfolgt ein Bewegen eines Sensorkopfs mit dem Metalloxid-Gassensor und/oder ein Bewegen eines Probengefäßes für die zu vermessende Probe in eine vorgesehene Messposition. In der Messposition ist der Sensorkopf oberhalb des Probengefäßes angeordnet.

In einem Schritt S12 wird ein initialer, vorbestimmter Sensorparameter ausgewählt und ein Ansteuern des Metalloxid-Gassensors mit dem wenigstens einen vorbestimmten Sensorparameter durchgeführt. Die Sensorparameter können Temperaturdaten, zeitlich veränderte Temperaturdaten oder Temperaturkurven sein, um den Gassensor auf eine gewünschte Messtemperatur einzustellen, bevorzugt aufzuheizen.

In einem Schritt S14 wird die Messung eines in der Probe im Probengefäß enthaltenen Stoffs durchgeführt, es werden also Messdaten mit dem Sensor aufgenommen. Die erzeugten Messdaten werden in einem Schritt S16 aufbereitet und ausgewertet. Dabei können die von dem Sensor zur Verfügung gestellten Messdaten beispielsweise in einer Auswerteeinheit ausgewertet werden.

In einem Schritt S18 werden Korrelationen zwischen den Messdaten und den Sensorparametern sowie dem Stoff der zu vermessenden Probe unter Anwendung einer Auswertungsmethode, die vorzugsweise eine Regressionsmethode und/oder eine Diskriminanzanalyse umfasst, ermittelt. Aus diesen Korrelationen lassen sich Einstellungen für weitere Sensorparameter ermitteln. Ergänzend oder alternativ kann ein Ermitteln von Korrelationen zwischen den Messdaten und der Analysestrategie und dem Stoff der zu vermessenden Probe unter Anwendung einer Auswertungsmethode erfolgen.

Ein Schritt S20 sieht ein Optimieren der Sensorparameter basierend auf den aufbereiteten Messdaten vor, um eine schnellere Anpassung des Metalloxid-Sensors oder anderer Metalloxid-Sensoren zu ermöglichen. Ergänzend oder alternativ kann ein Optimieren der Analysestrategie basierend auf den aufbereiteten Messdaten erfolgen, um eine schnellere Anpassung der Auswerteeinheit oder anderer Auswerteeinheiten zu ermöglichen.

Das Verfahren zeichnet sich dadurch aus, dass die Aufbereitung und Auswertung der Messdaten und die Anpassung und Optimierung der Sensorparameter und/oder der Analysestrategie während oder nach dem Messbetrieb erfolgt. Auf diese Weise ist eine automatisierte Sammlung von Sensorparametern möglich, die zum einen sehr schnell und gezielt erfolgen und zum anderen über eine lange Zeitdauer durchgeführt werden kann. Insbesondere, wenn die einzelnen Sensoren an einem Sensorkopf zusammengefasst werden und gleichzeitig mit mehreren Sensoren Stoffe der einzelnen Proben detektiert werden können, kann innerhalb relativ kurzer Zeit eine Vielzahl von Daten erzeugt werden. Beim Einsatz von KI-Einheiten ist dies notwendig, da die KI-Einheiten und das automatisierte Lernen, das auf einem evolutionären Lernen oder einem bestärkenden Lernen basieren kann, eine Vielzahl von Trainingsdaten und Messdaten benötigen.

Die KI-Einheit ist dazu ausgebildet, aus einer vorhergegangenen Anpassung zu lernen und ist vorzugsweise dazu ausgebildet, Trainingsdaten für andere Metalloxid-Sensoren und/oder andere Auswerteeinheiten zu erstellen.

Die Optimierung basiert auf mehreren Messungen mehrerer Proben und wird in einem Schritt S22 beendet, wenn ein vorgegebenes Abbruchkriterium, also eine maximale Anzahl von Messungen und/oder ein Gütekriterium erreicht wird.

Mittels des hier gezeigten Verfahrens und der vorgestellten Ausführungsform der Vorrichtung ist es möglich, einen "Sensor-Trainer" zu erzeugen und adaptive Modelle für die Steuerung von Gassensoren zur Adaption dieser Sensoren für die Erkennung unterschiedlicher Gasproben zu schaffen. Die Trainingsdaten, die die Sensorparameter für die Sensoren umfassen, werden automatisiert aufgenommen, aufbereitet und mit KI-Algorithmen einer KI-Einheit analysiert. Darüber hinaus ist es möglich, eine Visualisierung des Prozesses und der enthaltenen Daten über eine Webschnittstelle zu realisieren.

Die verwendeten Algorithmen der KI-Einheit können unabhängig von den Sensortypen und Sensorarten verwendet werden. Zusätzlich können Störungen der Umwelt gesteuert einbezogen werden, wenn dies gewünscht ist. Alternativ können diese Störungen vermieden werden, indem die Vorrichtung durch das Gehäuse von der Außenwelt entkoppelt ist.

Vorteilhaft an der erfindungsgemäßen Vorrichtung ist die Verwendung eines Sensorkopfs, in dem mehrere Sensoren platziert und angeordnet sein können. Auf diese Weise lassen sich automatisiert mehrere Sensoren gleichzeitig ansteuern, sodass gleichzeitig mehrere Messungen erfolgen können.

Durch die Verwendung eines abgeschlossenen Gehäuses ist es möglich, Störungen, die durch die Umgebungsluft beispielsweise im Labor verursacht werden können, zu vermeiden. Beispielsweise kann ein geringer Überdruck in dem Gehäuse erzeugt werden, um den Einfluss der Umgebung weiter zu reduzieren. Auch kann kontinuierlich Luft in das Gehäuse eingeführt werden. Die Luft in dem Gehäuse kann also getauscht werden, beispielsweise durch Null-Luft, Laborluft oder auch Außenluft. Auf diese Weise ist ein störungsfreier Messablauf zum Sammeln der Messdaten möglich, aus denen die Sensorparameter ermittelt werden. So lassen sich Sensordaten sammeln, die mit unterschiedlicher Nähe zu realen Bedingungen erprobt werden können. Durch den Einsatz der KI-Einheit für die Sensoren lassen sich auch unterschiedliche Umgebungen adaptieren und anpassen.

Die Verwendung des geschlossenen Gehäuses hat neben der Entkopplung der äußeren Umwelteinflüsse den Vorteil, dass eine bessere Kontrolle der Temperatur und Luftfeuchtigkeit während des Messbetriebs erfolgen kann. Dies wird durch regelbare Heizplatten oder Wärmeplatten unterhalb der Probengefäße unterstützt.

Die Erfindung wurde anhand der Zeichnungen und der Beschreibung umfassend beschrieben und erklärt. Die Beschreibung und Erklärung sind als Beispiel und nicht einschränkend zu verstehen. Die Erfindung ist nicht auf die offenbarten Ausführungsformen beschränkt. Andere Ausführungsformen oder Variationen ergeben sich für den Fachmann bei der Verwendung der vorliegenden Erfindung sowie bei einer genauen Analyse der Zeichnungen, der Offenbarung und der nachfolgenden Patentansprüche.

In den Patentansprüchen schließen die Wörter "umfassen" und "mit" nicht das Vorhandensein weiterer Elemente oder Schritte aus. Der undefinierte Artikel "ein" oder "eine" schließt nicht das Vorhandensein einer Mehrzahl aus. Ein einzelnes Element oder eine einzelne Einheit kann die Funktionen mehrerer der in den Patentansprüchen genannten Einheiten ausführen. Ein Element, eine Einheit, eine Vorrichtung und ein System können teilweise oder vollständig in Hard- und/oder in Software umgesetzt sein. Die bloße Nennung einiger Maßnahmen in mehreren verschiedenen abhängigen Patentansprüchen ist nicht dahingehend zu verstehen, dass eine Kombination dieser Maßnahmen nicht ebenfalls vorteilhaft verwendet werden kann. Ein Computerprogramm kann auf einem nichtflüchtigen Datenträger gespeichert/vertrieben werden. Ein Computerprogramm kann zusammen mit Hardware und/oder als Teil einer Hardware vertrieben werden, beispielsweise mittels des Internets oder mittels drahtgebundener oder drahtloser Kommunikationssysteme. Bezugszeichen in den Patentansprüchen sind nicht einschränkend zu verstehen.

## Patentansprüche

1. Vorrichtung (10) zum automatisierten Durchführen von Messungen und Sammeln von Messdaten und zum Erzeugen eines optimierten Sensorparameters für einen Metalloxid-Sensor (23) und/oder einer optimierten Analysestrategie einer Auswerteeinheit (70) zur Bestimmung eines in einer bekannten Probe enthaltenen Stoffs umfassend:
einen Sensorkopf (20) mit wenigstens einem Metalloxid-Sensor (23), der mit dem Sensorparameter angesteuert wird, wobei der Sensorparameter den Metalloxid-Sensor (23) zum Erfassen eines oder mehrerer spezifischer Stoffe in Proben konfiguriert;
zwei oder mehrere Probengefäße (30) für zu untersuchende bekannte Proben;
eine Bewegungseinheit (40) zum Bewegen des Sensorkopfs (20) und/oder eines Probengefäßes (30) in eine Messposition, in der der Sensorkopf (20) oberhalb eines der Probengefäße (30) angeordnet ist;
eine Steuereinheit (50) zum Ansteuern des Metalloxid-Sensors (23) mit dem Sensorparameter;
eine Auswerteeinheit (70) zum Auswerten der von dem Metalloxid-Sensor (23) während einer Messung gemessenen Messdaten;
eine KI-Einheit (80) zum Anpassen und/oder Optimieren des Sensorparameters basierend auf der bekannten Probe und aktuellen Messdaten und dem Sensorparameter;
wobei
die KI-Einheit (80) dazu ausgebildet ist, einen auf den Messdaten und den Sensorparametern basierenden Fitnesswert zu ermitteln, und eine Optimierung der Sensorparameter basierend auf den Messdaten und dem Fitnesswert durchzuführen;
die Anpassung und/oder Optimierung der Sensorparameter während der Messung oder nach einem Messzyklus erfolgt; und
die KI-Einheit (80) dazu ausgebildet ist, aus Messdaten früherer Messungen, den aktuellen Messdaten und/oder den verwendeten Sensorparametern einen verbesserten Sensorparameter für den Metalloxid-Sensor (23) zu generieren und/oder eine verbesserte Analysestrategie für die Auswerteeinheit (70) zu bestimmen.

2. Vorrichtung (10) nach Anspruch 1, **dadurch gekennzeichnet, dass** die KI-Einheit (80) eine optimierte Anpassung der Sensorparameter vornimmt, wobei die Anpassung durch Auswahl vorhandener und passender Sensorparameter aus einem Sensorparametersatz oder einer Datenbank oder durch Erzeugen neuer Sensorparameter erfolgt, wobei bevorzugt die Anpassung basierend auf vorhergehenden Anpassungen eines vorhergehenden Messzyklus oder einer vorherigen Messung oder auf Anpassungen von Sensorparametern von Sensoren für andere Stoffe erfolgen kann.

3. Vorrichtung (10) nach einem der vorhergehenden Ansprüche, **dadurch gekennzeichnet, dass** die Vorrichtung (10) eine Heizplatte (34), die wenigstens eines der Probengefäße (30) heizt, bevorzugt eine Heizplatte (34) für jedes der Probengefäße (30), umfasst, wobei bevorzugt die Heizplatte (34) von der Steuereinheit (50), der Auswerteeinheit (70) oder der KI-Einheit (80) angesteuert und deren Heizleistung eingestellt wird, wobei die Heizleistung der Heizplatte (34) als Sensorparameter mittels der KI-Einheit (80) optimierbar ist.

4. Vorrichtung (10) nach einem der vorhergehenden Ansprüche, **dadurch gekennzeichnet, dass** die Steuereinheit (50) dazu ausgebildet ist, den Sensor mittels eines Sensorparameters in Form einer Temperaturkurve, einer Temperatur und/oder einer Dauer einer Aufheizphase bis zum Erreichen einer vorgegebenen Temperatur anzusteuern, wobei bevorzugt der Sensorparameter zusätzlich Informationen zu der Umgebung, dem Sensortyp, zu Haltezeiten für bestimmte Temperaturen, zu Abkühlphasen der Proben oder der Messdauer je Probe umfasst.

5. Vorrichtung (10) nach einem der vorhergehenden Ansprüche, **dadurch gekennzeichnet, dass** die KI-Einheit (80) dazu ausgebildet ist, den Fitnesswert basierend auf einem Trennbarkeitswert, auf ausgewerteten Messdaten und vorzugsweise auf Referenzdaten zu berechnen, wobei die ausgewerteten Messdaten aus den Messdaten mittels einer Analysestrategie, insbesondere bevorzugt umfassend Datenaufbereitung, Merkmalsextraktion und -reduktion, Transformation und/oder Klassifikation, gewonnen werden und wobei die Referenzdaten aus einer Referenzmessung oder aus einer Referenzdatenbank mit sensortypspezifischen Referenzdaten erzeugt werden.

6. Vorrichtung (10) nach einem der vorhergehenden Ansprüche, **dadurch gekennzeichnet, dass** die KI-Einheit (80) die Anpassung der Sensorparameter und/oder die Anpassung der Analysestrategie mittels eines evolutionären Lernens, mittels eines bestärkenden Lernens, mittels Entscheidungsbäumen und/oder mittels eines künstlichen neuronalen Netzes durchführt.

7. Vorrichtung (10) nach einem der vorhergehenden Ansprüche, **dadurch gekennzeichnet, dass** die KI-Einheit (80) dazu ausgebildet ist, Rückschlüsse von Abhängigkeiten zwischen Temperatursteuerkurve, Sensortyp, Heiztemperatur, Arbeitstemperatur, zu messender Probe, umfassten Stoffen in der Probe, Analysestrategie und/oder optional Temperatur der Heizplatte (34) zu ziehen, um einen optimierten Sensorparameter zur Steuerung des Metalloxid-Sensors (23) oder für andere Metalloxid-Sensoren (23) und/oder eine optimierte Analysestrategie für die Auswerteeinheit (70) oder andere Auswerteeinheiten (70) zu generieren.

8. Vorrichtung (10) nach einem der vorhergehenden Ansprüche, **dadurch gekennzeichnet, dass** der Sensorkopf (20), die zwei oder mehreren Probengefäße (30) und die Bewegungseinheit (40) in einem Gehäuse (90) mit einer verschließbaren Öffnung (38) angeordnet sind, um die Messungen in einer definierten Umgebung auszuführen, wobei ein Öffnungsgrad der Öffnung (38) mittels der Steuereinheit (50) einstellbar und mittels der KI-Einheit (80) als Sensorparameter optimierbar ist.

9. Vorrichtung (10) nach einem der vorhergehenden Ansprüche, **dadurch gekennzeichnet, dass** die Steuereinheit (50) dazu ausgebildet ist, vor einer Messung einer Probe eine Referenzmessung mit Luft, Reinluft, Umgebungsluft oder einer Referenzprobe durchzuführen.

10. Vorrichtung (10) nach einem der vorhergehenden Ansprüche, **dadurch gekennzeichnet, dass** die Vorrichtung (10) mobil ist und dazu eingerichtet ist, um die Metalloxid-Sensoren (23) in Einsatzumgebungen für die jeweilige Anwendung optimal einzustellen und die Einflüsse der jeweiligen Einsatzumgebung widerzuspiegeln, wobei bevorzugt die Vorrichtung (10) eine Energieversorgungseinheit umfasst, um die mobile Vorrichtung (10) an eine vorhandene Energieversorgung anzuschließen oder autark zu betreiben.

11. Verfahren zum automatisierten Durchführen von Messungen und Sammeln von Messdaten und zum Erzeugen von optimalen Sensorparametern eines Metalloxid-Sensors (23) und/oder einer optimierten Analysestrategie einer Auswerteeinheit zur Bestimmung eines in einer Probe enthaltenen Stoffs oder zur Bestimmung von Trainingsdaten eines derartigen Metalloxid-Sensors (23), umfassend die folgenden Schritte:
- Bewegen (S10) eines Sensorkopfs (20) mit einem Metalloxid-Sensor (23) und/oder eines Probengefäßes (30) für die zu vermessende Probe in eine Messposition, in der der Sensorkopf (20) oberhalb des Probengefäßes (30) angeordnet ist;
- Auswählen (S12) eines initialen, vorbestimmten Sensorparameters;
- Ansteuern (S12) des Metalloxid-Sensors (23) mit dem vorbestimmten Sensorparameter;
- Messen (S14) eines Stoffs, der in der Probe in dem Probengefäß (30) umfasst ist;
- Aufnehmen (S14) von Messdaten mit dem Metalloxid-Sensor (23) und Auswerten der aufgenommenen Messdaten;
- Aufbereiten (S16) der Messdaten, wobei vorzugsweise eine Datenbereinigung, -transformation, -korrektur und Berücksichtigung von Umgebungsdaten und/oder eine Dimensionalitätsreduktion erfolgt;
- Ermitteln von Korrelationen (S18) zwischen den Messdaten und den Sensorparametern und dem Stoff der zu vermessenden Probe unter Anwendung einer Auswertungsmethode, die vorzugsweise eine Regressionsmethode und/oder eine Diskriminanzanalyse umfasst;
- Optimieren (S20) der Sensorparameter basierend auf den aufbereiteten Messdaten, um eine schnellere Anpassung des Metalloxid-Sensors (23) oder anderer Metalloxid-Sensoren (23) zu ermöglichen,
- ergänzend oder alternativ Ermitteln von Korrelationen (S18) zwischen den Messdaten und der Analysestrategie und dem Stoff der zu vermessenden Probe unter Anwendung einer Auswertungsmethode;
- ergänzend oder alternativ Optimieren (S20) der Analysestrategie basierend auf den aufbereiteten Messdaten, um eine schnellere Anpassung des Metalloxid-Sensors (23) oder anderer Metalloxid-Sensoren (23) zu ermöglichen,
- wobei die Aufbereitung und Auswertung der Messdaten und die Anpassung und Optimierung der Sensorparameter und/oder der Analysestrategie während oder nach dem Messbetrieb erfolgt und wenigstens teilweise von einer KI-Einheit (80) durchgeführt wird, die dazu ausgebildet ist, aus einer vorhergegangenen Anpassung zu lernen und vorzugsweise dazu ausgebildet ist, Trainingsdaten für andere Metalloxid-Sensoren (23) und/oder andere Auswerteeinheiten zu erstellen, und
- wobei die Optimierung auf mehreren Messungen mehrerer Proben basiert, und die Optimierung beendet (S22) wird, wenn ein vorgegebenes Abbruchkriterium erfüllt ist.

12. Computerprogrammprodukt mit Programmcode zum Durchführen der Schritte des Verfahrens nach Anspruch 11, wenn der Programmcode auf einem Computer ausgeführt wird.

13. Speichermedium zur Verwendung in einer Steuereinheit zum Steuern eines Metalloxid-Sensors (23), wobei ein nach dem Verfahren nach Anspruch 11 oder mittels einer Vorrichtung nach einem der Ansprüche 1 bis 10 optimierter Sensorparameter auf dem Speichermedium gespeichert ist und zur Steuerung des Metalloxid-Sensors (23) verwendet wird.

14. KI-Einheit (80) für eine Vorrichtung (10) nach einem der Ansprüche 1 bis 10 mit: einer Schnittstelle zum Austauschen von Daten zwischen der KI-Einheit (80) und der Steuereinheit (50) und/oder der Auswerteeinheit (70) der Vorrichtung (10), wobei die KI-Einheit (80) dazu ausgebildet ist, basierend auf von der Vorrichtung (10) empfangenen Messdaten wenigstens eine Einheit der Vorrichtung (10) zu konfigurieren, um ein Messverfahren der Vorrichtung (10) zu optimieren.

15. KI-Einheit (80) nach dem vorstehenden Anspruch, wobei die KI-Einheit (80) dazu ausgebildet ist, einen Sensorparameter zu optimieren, um eine Steuereinheit (50) der Vorrichtung zu optimieren; und/oder wobei die KI-Einheit (80) dazu ausgebildet ist, eine Analysestrategie zu optimieren, um eine Auswerteeinheit (70) der Vorrichtung zu optimieren.
